# EUROPEAN PATENT APPLICATION

(11) **EP 4 656 661 A1**
(43) Date of publication of application: **03.12.2025**
(21) Application number: 24315253.5
(22) Date of filing: 29.05.2024
(51) Int. Cl.: C08F 293/00, C08F 290/06, A61C 13/00, A61K 6/896, B33Y 10/00, C08F 212/08, C08F 220/14

(54) **BLOCK COPOLYMERS SUITABLE AS IMPACT MODIFIERS, PROCESS FOR THEIR PREPARATION AND POLYMERIZABLE MATERIALS COMPRISING THEM**

(71) Applicant: Ivoclar Vivadent AG, 9494 Schaan (LI); Centre National de la Recherche Scientifique, 75794 Paris Cedex 16 (FR); Aix-Marseille Université, 13007 Marseille (FR)
(72) Inventor: CATEL, Yohann, 9475 Sevelen (CH); LAMPARTH, Iris, 9472 Grabs (CH); RIST, Kai, 6800Feldkirch (AT); OMERAGIC, Sadini, 9466 Sennwald (CH); PHAN, Trang, 13009 Marseille (FR); GIGMES, Didier, 13190 Allauch (FR); HO, The Hien, Ho Chi Min City (VN); AYHAN, Mehmet Menaf, 1130 Wien (AT)
(74) Representative: Uexküll & Stolberg

(57) **Abstract**

Block copolymer according to Formula 1 and methods for producing the same, 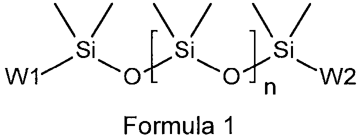 wherein n is a value of from 5 to 250; W1 is a group according to Formula 2, which is attached to the Si atom of Formula 1 via X:

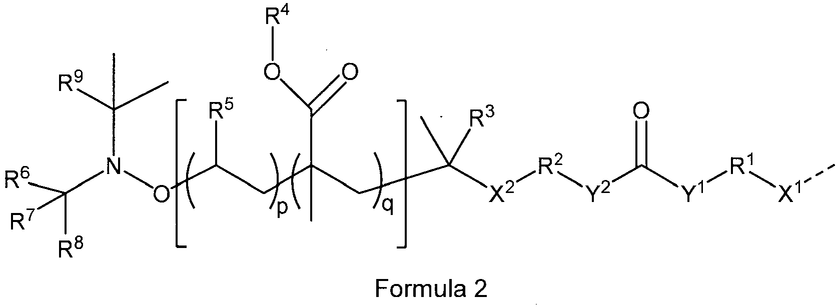

The block copolymers are suitable to improve fracture toughness of radically polymerizable compositions. These compositions are particularly suitable as dental materials.

## Description

The present invention relates to block copolymers suitable as impact modifiers, in particular for radically polymerizable compositions, which are especially suitable as dental material for the production of dental shaped bodies, such as denture bases and teeth, splints (bite splints), temporary crowns, and orthodontic appliances, by additive processes. The invention also relates to methods for the production of such block copolymers.

In recent years, additive manufacturing processes have found widespread use in industry and dentistry for the production of shaped bodies. In dentistry, stereolithography and Digital Light Processing (DLP) are mostly used to produce dental bodies such as denture bases, denture teeth, splints, dental composites, models, and temporary crowns. In this method, a projector is used as a light source to cure a resin layer by layer.

Conventional denture bases, are mainly made by thermal polymerization of a mixture of poly(methyl methacrylate) (PMMA) powder with a methyl methacrylate (MMA)-based liquid. Due to the low reactivity and high volatility of MMA, conventional MMA-based denture base materials are hardly suitable for 3D printing. Di(meth)acrylate mixtures are used to circumvent this problem. Dimethacrylate-based materials are characterized by high reactivity and good mechanical properties, but their polymerization results in the formation of brittle polymer networks. Such networks exhibit a significantly lower fracture toughness than PMMA materials that are produced according to the traditional workflow. These brittle materials are not suitable for many clinical indications.

To improve the fracture toughness of dimethacrylate-based materials, rubber particles, core-shell particles, thermoplastic particles, or silica nanoparticles are added as impact modifiers or tougheners.

WO 2014/078537 A1 discloses resin mixtures for the production of dental shaped bodies by 3D printing processes based on mono- and multifunctional methacrylates, which contain silicone acrylate-based impact modifiers with a core-shell structure for improving the impact resistance and fracture toughness.

US 2018/0000570 A1 relates to building materials based on mono- and multifunctional (meth)acrylates for the additive production of dental components. The building materials contain rubber particles based on silicone acrylic with a core-shell structure as impact modifiers and oligomers, which are prepared by reacting trimethyl 1,6-diisocyanate, bisphenol A propoxylate and 2-hydroxyethyl methacrylate (HEMA). The cured components are said to have good mechanical and physical properties as well as a good biocompatibility.

US 2019/0053883 A1 discloses dental components produced by additive processes which have at least two layers of building materials with different compositions. One layer is formed by a material which contains oligomers, which are obtained by reacting intermediate products having terminal isocyanate groups with hydroxyl-based methacrylates, a polymerizable acrylic compound and an impact modifier. At least one further layer is formed by a material which contains a urethane monomer, a glycol dimethacrylate and filler. The combination of materials with different mechanical and physical properties is said to be advantageous for adapting the components to different requirements.

EP 3 564 282 A1 discloses curable compositions for high-temperature photopolymerization processes which contain an oligomeric urethane dimethacrylate as glass transition temperature modifier, a (poly)carbonate-(poly)urethane dimethacrylate as toughness modifier and optionally core-shell particles. They are said to have good thermomechanical properties and good biocompatibility and to be suitable for the production of orthodontic appliances.

WO 2014/077848 A1 discloses compositions for three-dimensional (3D) printing comprising a liquid, curable, cross-linkable monomer together with solid thermoplastic particles of a size ranging from about 200 nm to about 50 µm and a photoinitiator. These compositions are claimed to be of high strength and toughness.

Although fracture toughness can be improved by the addition of particulate impact modifiers, these modifiers have certain disadvantages. Thermoplastic particles and rubber particles have to be added in significant amounts. This restricts processing due to the high viscosity of the materials, leads to lower monomer conversion and thus to poorer mechanical properties. Core-shell-particles are active at lower concentrations, but it is difficult to achieve a homogeneous dispersion as they tend to agglomerate. In addition, particulate additives generally increase the opacity of the materials due to light scattering which is disadvantageous for stereolithographic processes.

It was recently shown that certain block copolymers can improve the fracture toughness of radically polymerizable materials. EP 4 085 893 A1 discloses materials that contain at least one di- or triblock copolymer in combination with a urethane dimethacrylate and a monomethacrylate that exhibit high fracture toughness. The block copolymers contain an A block that is miscible with the monomer matrix and an immiscible B block. The A block is preferably a poly(meth)acrylate, polylactone, phenylenoxid or polyalkylenoxide oligomer, whereas the B block is preferably a polysiloxane, polyvinyl, polyalkene or polydiene oligomer. By use of these di- or triblock copolymers, the disadvantages associated with particulate impact modifiers could be overcome.

It is an object of the present invention to provide additional means for improving the fracture toughness of radically polymerizable resins which do not cause the above-mentioned disadvantages. It is a further object of the invention is to provide radically polymerizable materials with high fracture toughness and good mechanical properties, especially under wet conditions, which are particularly suitable as dental materials, especially for the manufacture of dental restorations and dental prosthesis by 3D printing.

These objects are achieved by block copolymers (BCP) according to Formula 1, 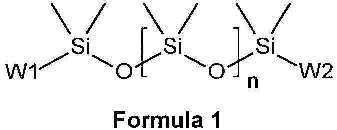 wherein
- n: is a value of from 5 to 250, preferably from 10 to 200, more preferably from 15 to 150;
- W1: is a group according to Formula 2, which is attached to the Si atom of Formula 1 via X¹, as indicated by the dashed line, 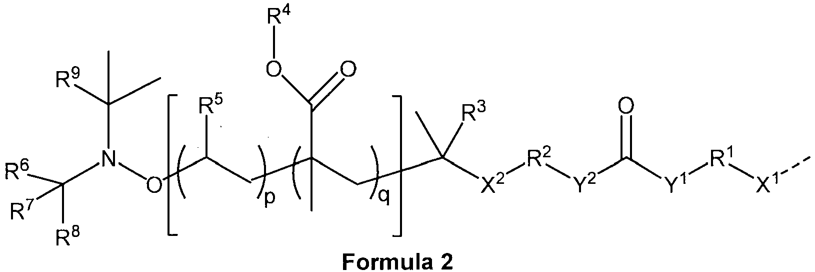
wherein
- X¹: is CH₂ or an O atom;
- X²: is a -C(O)O- group or is absent;
- Y¹: is absent, an O atom, a NR²¹ group in which R²¹ is a hydrogen atom or a branched or a linear C₁-C₁₂ alkyl group, preferably a hydrogen atom or a C₁-C₈ alkyl group, and more preferably a hydrogen atom or a C₁-C₆ alkyl group, whereby Y¹ is absent if Y² is an O atom;
- Y²: is an O atom or is absent, whereby Y² is absent if Y¹ is an O atom or a NR²¹ group;
- R¹: is a branched or linear C₁-C₁₆ alkanediyl group, preferably a branched or linear C₁-C₁₂ alkanediyl group, and more preferably a branched or linear C₁-C₆ alkanediyl group, that is interrupted by one or more O atoms and/or -C(O)O- groups and is preferably uninterrupted;
- R²: is absent or a branched or a linear C₁-C₁₀ alkanediyl group, preferably a branched or linear C₁-C₈ alkanediyl group, and more preferably a branched or linear C₁-C₆ alkanediyl group, that is uninterrupted or interrupted by one or more, preferably one, O atoms and/or -C(O)O- groups, whereby R² cannot be absent if X² is a -C(O)O- group;
- R³: is hydrogen, a methyl, nitrile or -(C=O)OR¹⁰ group, preferably a methyl or nitrile group, and R¹⁰ is a branched or a linear C₁-C₁₂ alkyl group, preferably a branched or a linear C₁-C₈ alkyl group, and more preferably a branched or a linear C₁-C₆ alkyl group;
- R⁴: is a branched or preferably linear C₁-C₁₂ alkyl group, preferably a branched or preferably linear C₁-C₁₀ alkyl group, and more preferably a branched or preferably linear C₁-C₈ alkyl group, that is uninterrupted or interrupted by one or more, preferably 1 to 3, and more preferably 1 to 2, S atoms, O atoms, -C(O)O- groups and/or -C(O)NH- groups, and which is unsubstituted or substituted by one or more, preferably one, functional groups, preferably OH, COOH and/or halogen;
or
a C₄-C₁₆ cycloaliphatic group that is unsubstituted or substituted by one or more, preferably 1 to 5, and more preferably 1 to 3, substituents selected from branched or preferably linear C₁-C₂₀ alkyl groups, preferably branched or preferably linear C₁-C₁₂ alkyl groups, more preferably branched or preferably linear C₁-C₆ alkyl groups, and that is uninterrupted or interrupted by one or more, preferably 1 to 4, and more preferably 1 to 2, S atoms, O atoms, -C(O)O- groups and/or C(O)NH- groups;
or
a C₄-C₁₀ aryl or heteroaryl group which is unsubstituted or substituted by one or more, preferably 1 to 4, and more preferably 1 to 2, substituents selected from branched or preferably linear C₁-C₂₀ alkyl groups, -O-C₁-C₁₂ alkoxy groups, ester groups -C(O)O-R¹¹, and/or acyl groups -C(O)-R¹², wherein R¹¹ and R¹² are each independently of one other a branched or preferably linear C₁-C₂₀ alkyl group, a C₆-C₁₀ aryl group or a C₇-C₂₀-alkylaryl group, and can be interrupted by one or more S atoms or O atoms and are preferably uninterrupted;
or
a C₅-C₂₀-alkylaryl or C₅-C₂₀-alkylheteroaryl group that is uninterrupted or interrupted by one or more, preferably 1 to 2, and more preferably 1 S atoms, O atoms, -C(O)O- groups and/or -C(O)NH- groups, and that is unsubstituted or substituted by one or more, preferably 1 to 4, and more preferably 1 to 2, substituents selected from C₁-C₂₀ alkyl groups, branched or preferably linear -O-C₁-C₁₂ alkoxy groups, branched or preferably linear ester groups -C(O)O-R¹³, and/or branched or preferably linear acyl groups -C(O)-R¹⁴, where R¹³ is a branched or preferably linear C₁-C₂₀ alkyl group, a C₆-C₁₀ aryl group or a C₇-C₂₀-alkylaryl group, and R¹⁴ is a branched or preferably linear C₁-C₂₀ alkyl group, a C₆-C₁₀ aryl group or a C₇-C₂₀-alkylaryl group;

- R⁵: is a phenyl group or a -(C=O)OR¹⁵ group in which
- R¹⁵: is a branched or preferably linear C₁-C₁₂ alkyl group, preferably a branched or preferably linear C₁-C₁₀ alkyl group, and more preferably a branched or preferably linear C₁-C₈ alkyl group, that is uninterrupted or interrupted by one or more, preferably 1 to 4, and more preferably 1 to 2, S atoms, O atoms, -C(O)O- groups, and/or -C(O)NH- groups, and that is unsubstituted or is substituted by one or more functional groups, preferably OH, COOH and/or halogen;
or
a C₄-C₁₆ cycloaliphatic group that is unsubstituted or substituted by one or more, preferably 1 to 5, and more preferably 1 to 3, substituents selected from branched or preferably linear C₁-C₂₀ alkyl groups, preferably from branched or preferably linear C₁-C₁₂ alkyl groups, more preferably from branched or preferably linear C₁-C₆ alkyl groups, and that is uninterrupted or interrupted by one or more, preferably 1 to 4, and more preferably 1 to 2, S atoms, O atoms, -C(O)O- groups and/or C(O)NH- groups;
or
a C₄-C₁₀ aryl or heteroaryl group that is unsubstituted or substituted by one or more, preferably 1 to 4, and more preferably 1 to 2, substituents which are selected from branched or preferably linear C₁-C₂₀ alkyl groups, branched or preferably linear -O-C₁-C₁₂ alkoxy groups, branched or preferably linear ester groups -C(O)OR¹⁶, and/or branched or preferably linear acyl groups -C(O)-R¹⁷, and that is uninterrupted or interrupted by one or more S atoms or O atoms, where R¹⁶ is a branched or preferably linear C₁-C₂₀ alkyl group, a C₆-C₁₀ aryl group or a C₇-C₂₀-alkylaryl group, and R¹⁷ is a branched or preferably linear C₁-C₂₀ alkyl group, a C₆-C₁₀ aryl group or a C₇-C₂₀-alkylaryl group,
or
a C₅-C₂₀-alkylaryl or C₅-C₂₀-alkylheteroaryl group that is uninterrupted or interrupted by one or more, preferably 1 to 2, and more preferably 1, S atoms, O atoms, -C(O)O- groups and/or -C(O)NH-groups, and that is unsubstituted or substituted by one or more, preferably 1 to 4, and more preferably 1 to 2, substituents selected from branched or preferably linear C₁-C₂₀ alkyl groups, branched or preferably linear O-C₁-C₁₂ alkoxy groups, branched or preferably linear ester groups -C(O)O-R¹⁸, and/or branched or preferably linear acyl groups -C(O)-R¹⁹, where R¹⁸ is a branched or preferably linear C₁-C₂₀ alkyl group, a C₆-C₁₀ aryl group or a C₇-C₂₀-alkylaryl group, and R¹⁹ is a branched or preferably linear C₁-C₂₀ alkyl group, a C₆-C₁₀ aryl group or a C₇-C₂₀-alkylaryl group;

- R⁶: is a hydrogen atom, a methyl group, or forms together with R⁹ a -CH₂-CHR²²-CH₂- group, wherein R²² is H or an OH group;
- R⁷: is a methyl group, a -CH(CH₃)₂ group or a -C(CH₃)₃ group;

- R⁸: is a methyl group, preferably a phenyl group or a P(O)(OR²⁰)₂ group in which R²⁰ is a branched or preferably linear C₁-C₁₂ alkyl group, preferably a branched or preferably linear C₁-C₆ alkyl group, and more preferably a branched or preferably linear C₁-C₄ alkyl group;
- R⁹: is a methyl group or forms together with R⁶ a -CH₂-CHR²²-CH₂- group, wherein R²² is H or an OH group;
- p: is a number of from 1 to 400, preferably from 1 to 200, more preferably from 1 to 140;
- q: is a number of from 0 to 400, preferably from 0 to 200, more preferably from 0 to 140; p + q is > 5;
- W2: is a group according to Formula 2, which is attached to the Si atom of Formula 1 via X¹, as indicated by the dashed line, or is a branched or preferably linear C₁-C₁₂ alkyl group, preferably a group according to Formula 2 or a branched or preferably linear C₁-C₆ alkyl group, and more preferably a group according to Formula II or a branched or preferably linear C₁-C₄ alkyl group.

The groups W1 and W2 comprise a polymeric block according to the following formula:

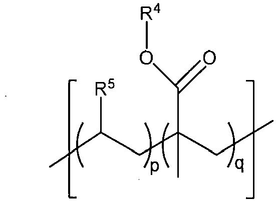

This block is an acrylate polymer (q = 0; R⁵ = -(C=O)OR¹⁵) or preferably a random copolymer block of a styrene (R⁵ = phenyl) or acrylate (R⁵ = -(C=O)OR¹⁵) repeating unit and a methacrylate repeating unit. The composition of the copolymer block is determined by the numbers p and q of the repeating units. p and q are average values and are usually determined by proton NMR.

The copolymer block comprises p + q monomer units. The ratio p/(p+q) of the monomer units is preferably within a range of from 0.01 to 0.5, more preferably from 0.02 to 0.2, most preferably from 0.04 to 0.15, if R⁵ is a phenyl group, and is preferably within a range of from 0.01 to 1.0, more preferably from 0.02 to 1.0, most preferably from 0.04 to 1.0, if R⁵ is a -(C=O)OR¹⁵ group.

If R⁵ is a phenyl group, p is most preferably a number of from 1 to 10, and q is most preferably a number of from 10 to 140. If R⁵ is a -(C=O)OR¹⁵ group, p is most preferably a number of from 6 to 140, and q is most preferably a number of from 0 to 140.

In one embodiment of the invention, R⁶ and R⁹ together form a -CH₂-CHR²²-CH₂-group. This group, together with the carbon atoms to which R⁶ and R⁹ are attached, forms a 6-membered N-heterocyclic ring as shown in Formula 2a:

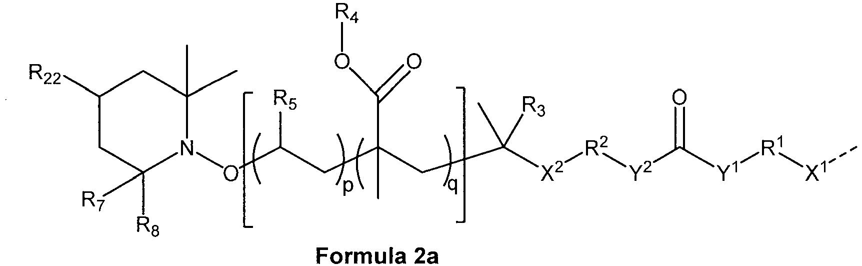

In Formula 2a, R²² is a hydrogen or a OH group and R⁷ and R⁸ are methyl groups. The other residues have the meaning defined above.

The block copolymers of Formula 1 are triblock copolymers of the ABA type if both W1 and W2 are a group of Formula 2, or diblock copolymers of the AB type if W2 is not a group of Formula 2. Triblock copolymers are preferred over diblock copolymers. If W2 is a group according to Formula 2, the variables of this group have the same meanings as in W1. However, as is well known to a skilled person, copolymer chains show a distribution of molar masses and are nonhomogeneous with regard to the distribution of the types of monomer units, so that W1 and W2 may differ in this respect.

Triblock copolymers of Formula 1 can be represented by Formula 3 and diblock copolymers by Formula 4:

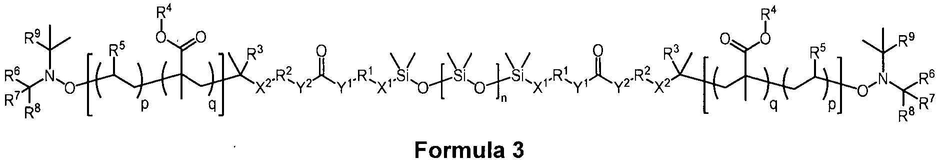

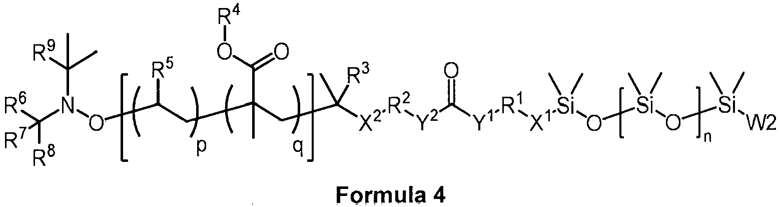

Triblock and diblock copolymers wherein R⁶ and R⁹ together form a -CH₂-CHR²²-CH₂-group and R⁷ and R⁸ are methyl groups can be represented by Formula 3a and Formula 4a, respectively:

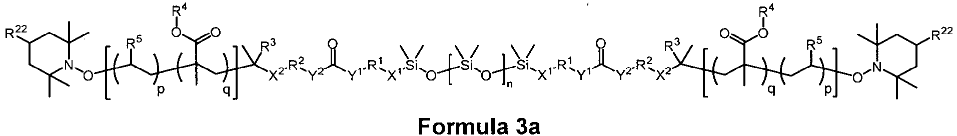

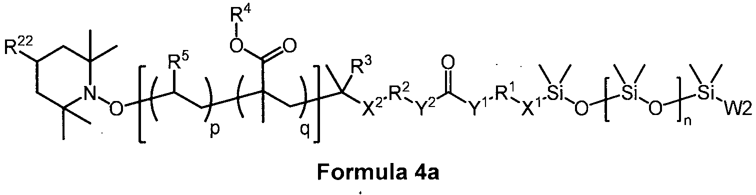

In Formulae 4 and 4a, W2 is a branched or preferably linear C₁-C₁₂ alkyl group, preferably a branched or preferably linear C₁-C₆ alkyl group, and more preferably a branched or preferably linear C₁-C₄ alkyl group.

All formulas shown herein extend only to those compounds which are compatible with the theory of chemical valence. The indication that a radical is interrupted e.g. by one or more oxygen atoms, is to be understood to mean that these atoms are inserted in each case into the carbon chain of the radical. These atoms are thus bordered on both sides by C atoms and cannot be terminal. C₁ radicals cannot be branched or interrupted.

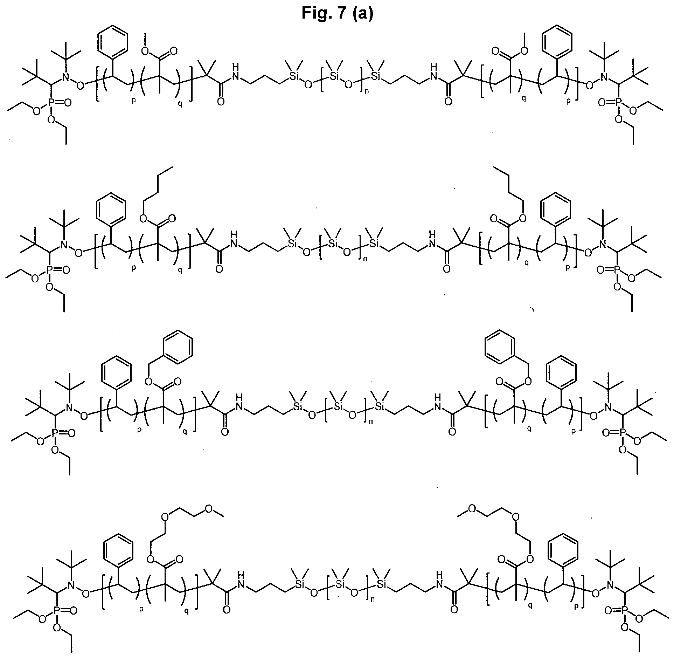

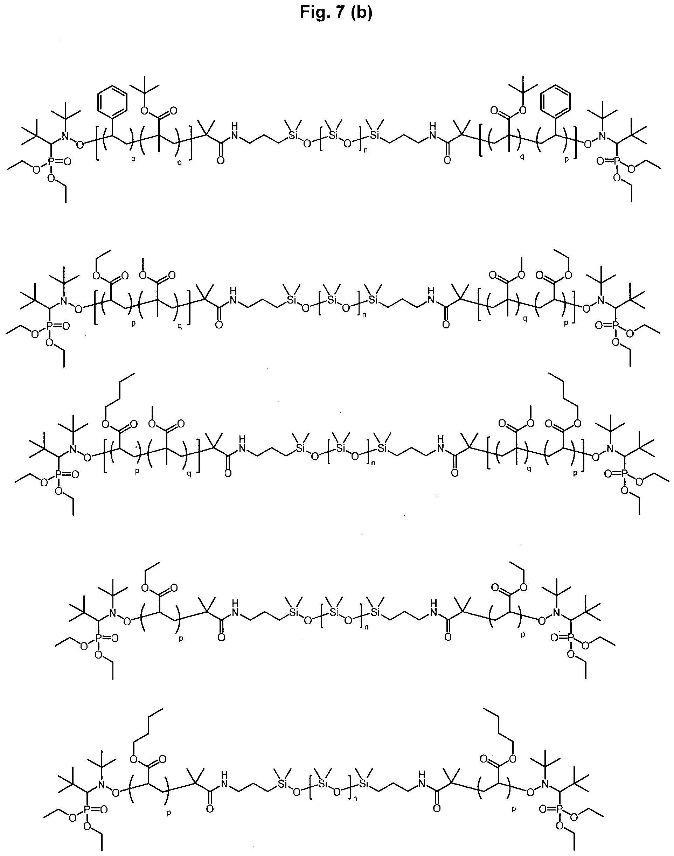

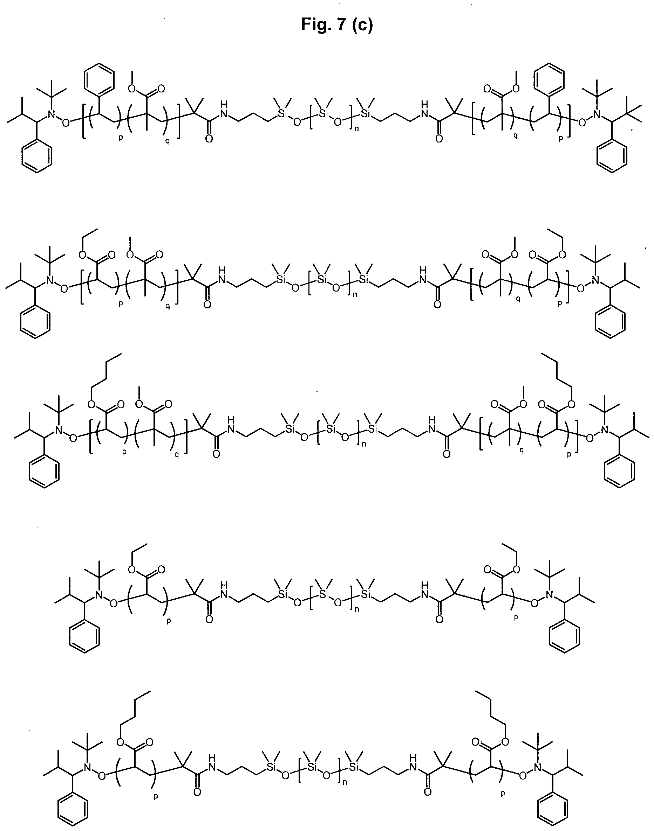

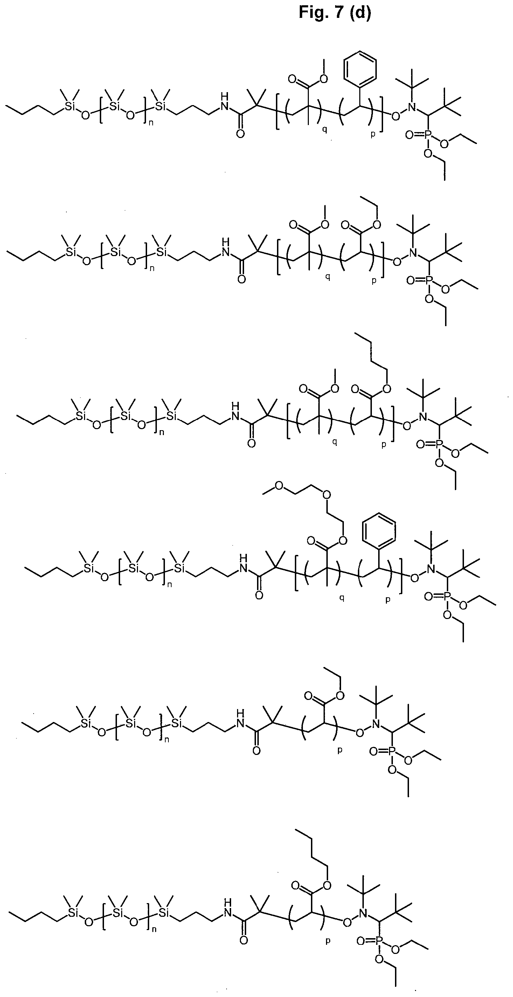

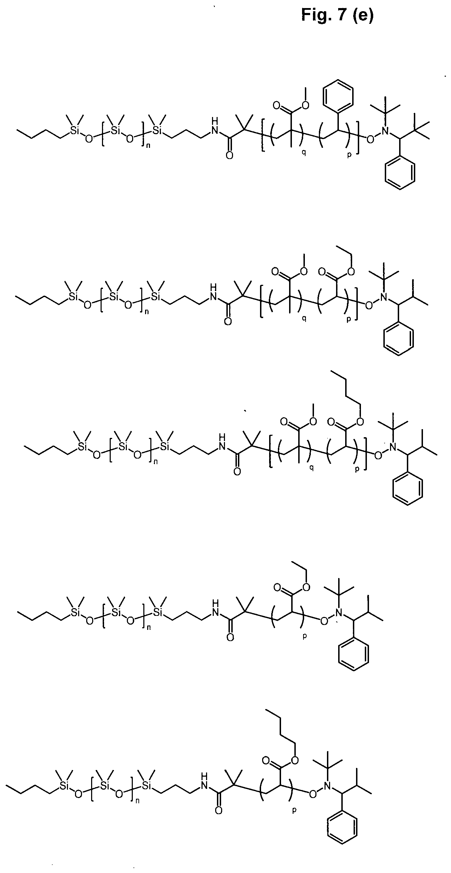

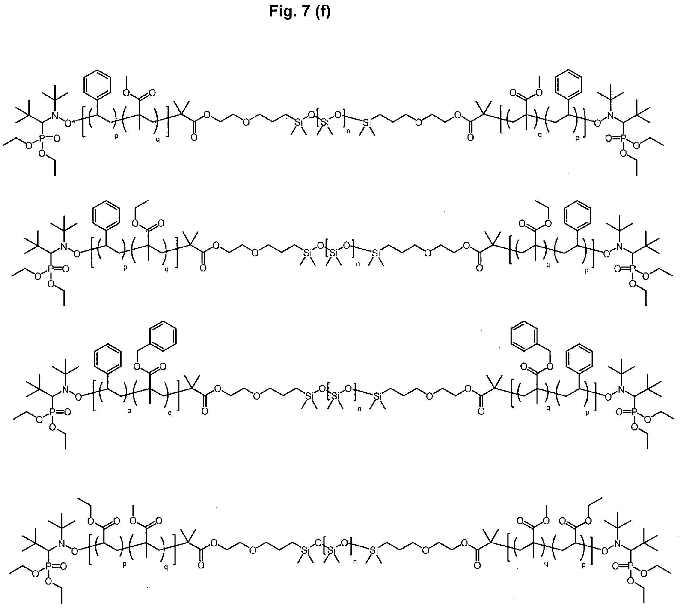

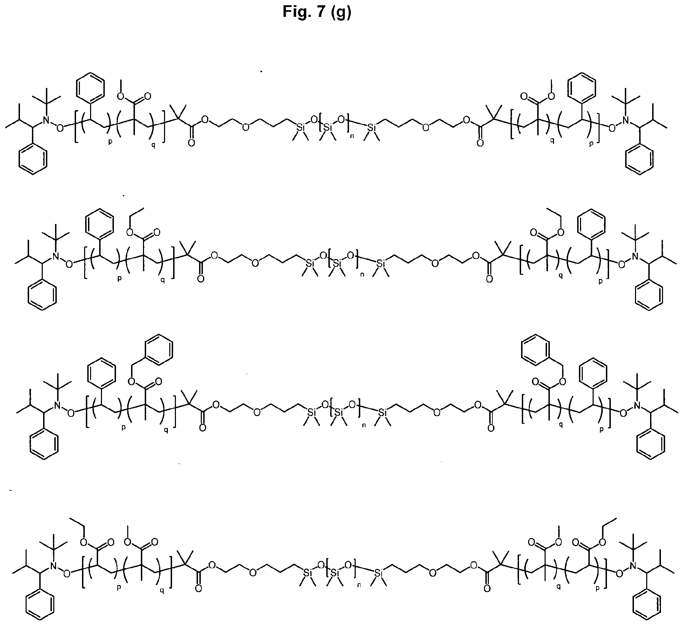

The preferred, particularly preferred and quite particularly preferred definitions given for the individual variables can be selected in each case independently of each other. Naturally, compounds in which all variables have the preferred, more preferred and most preferred definitions are particularly suitable in the sense of the invention. Particularly preferred block copolymers according to the invention are shown in Figure 7.

Particularly preferred block copolymers according to the invention are copolymers wherein the variables of Formula 1 have the following meanings:
- n: is a value of from 15 to 150;
- W1: is a group according to Formula 2, wherein
- X¹: is CH₂;
- X²: is absent;
- Y¹: is a NR²¹ group in which R²¹ is a hydrogen atom;
- Y²: is absent;
- R¹: is a linear C₁-C₄ alkanediyl group;
- R²: is absent;
- R³: is a methyl group;
- R⁴: is a linear C₁-C₈ alkyl group, that is uninterrupted or interrupted by 1 to 2, O atoms;
- R⁵: is a phenyl group or a -(C=O)OR¹⁵ group in which
- R¹⁵: is a branched or preferably linear C₁-C₈ alkyl group or a C₄-C₁₆ cycloaliphatic group that is unsubstituted or substituted by one or more, preferably 1 to 5, and more preferably 1 to 3, branched or preferably linear C₁-C₆ alkyl groups, or a C₄-C₁₀ aryl or heteroaryl group or a C₅-C₂₀-alkylaryl or C₅-C₂₀-alkylheteroaryl group
- R⁶: is a hydrogen atom;
- R⁷: is a -CH(CH₃)₂ group or a -C(CH₃)₃ group;
- R⁸: is a P(O)(OR²⁰)₂ group in which R²⁰ is a phenyl group or preferably a linear C₁-C₄ alkyl group, more preferably an ethyl group;
- R⁹: is a methyl group;
if R⁵ is a phenyl group, p and q have the following meanings:
- p: is a value of from 1 to 10;
- q: is a value of from 10 to 140;
- p + q: is > 5;
- p/(p+q): is a value of from 0.04 to 0.15;
if R⁵ is a -(C=O)OR¹⁵ group, p and q have the following meanings:
- p: is a value of from 6 to 140;
- q: is a value of from 0 to 140; p + q is > 5;
- p/(p+q): is a value of from 0.04 to 1.0;
- W2: is a branched or preferably linear C₁-C₄ alkyl group, or preferably is equal to W1.

Such block copolymers wherein W2 = W1 can be represented by the Formula 5:

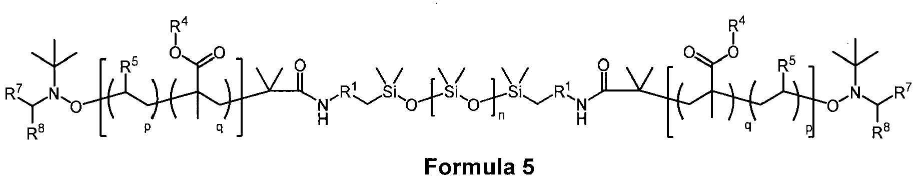

Most preferred block copolymers according to the invention are copolymers wherein the variables of Formula 1 have the following meanings:
- n: is a number of from 20 to 125;
- W1: is a group according to Formula 2, wherein
- X¹: is CH₂;
- X²: is absent;
- Y¹: is a NR²¹ group in which R²¹ is a hydrogen atom;
- Y²: is absent;
- R¹: is a linear C₁-C₄ alkanediyl group;
- R²: is absent;
- R³: is a methyl group;
- R⁴: is a linear C₁-C₈ alkyl group, that is uninterrupted or interrupted by 1 to 2, O atoms
- R⁵: is a phenyl group;
- R⁶: is a hydrogen atom;
- R⁷: is a -C(CH₃)₃ group;
- R⁸: is a P(O)(OR²⁰)₂ group in which R²⁰ is linear C₁-C₄ alkyl group, preferably an ethyl group;
- R⁹: is a methyl group;
- p: is a value of from 1 to 10;
- q: is a value of from 10 to 140;
- p/(p+q): is a value of from 0.04 to 0.15;
- W2: is equal to W1.

Such block copolymers can be represented by the Formula 6:

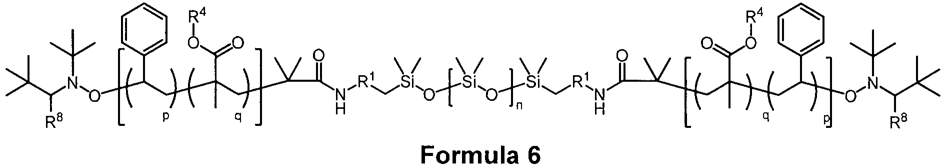

Block copolymers of Formula 1 are not known. A reason for this is possibly that such block copolymers are difficult to synthesize. The present invention also provides a new synthesis route that allows easy access to such block copolymers.

A key step in the synthesis of the block copolymers of Formula 1 is a nitroxide-mediated radical polymerization (NMP) using poly(dimethylsiloxane) (PDMS) alkoxyamines as macroinitiators.

The first step of the synthesis is the preparation of telechelic poly(dimethylsiloxane) (PDMS) oligomers according to Formula 7 (for the preparation of triblock copolymers) or 8 (for the preparation of diblock copolymers).

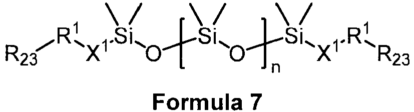

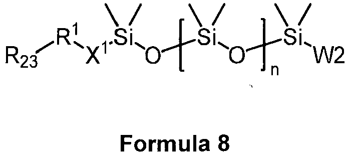

In which R²³ is a OH, a COOH or a NHR²¹ group, wherein R²¹ is a hydrogen atom or a branched or a linear C₁-C₁₂ alkyl group, preferably a hydrogen atom or a C₁-C₈ alkyl group, and more preferably a hydrogen atom or a C₁-C₆ alkyl group.

As an example, PDMS bearing two NH₂ end groups can be prepared by ring opening polymerization of octamethylcyclotetrasiloxane with the corresponding diamine:

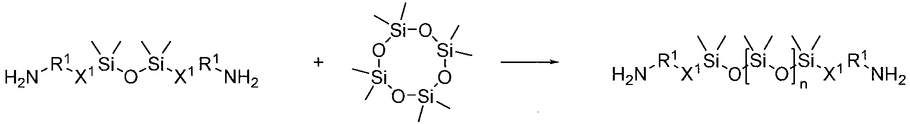

Hydroxyalkyl-terminated PDMS blocks (with X¹ = CH₂) can be prepared according to a similar pathway or by the platinum catalyzed reaction of an unsaturated alcohol with a di-SiH-functional polydimethylsiloxane:

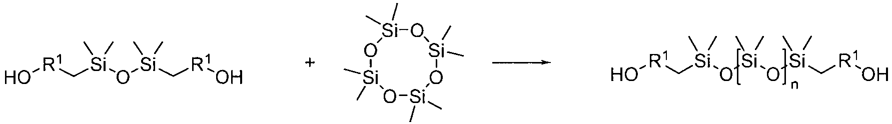

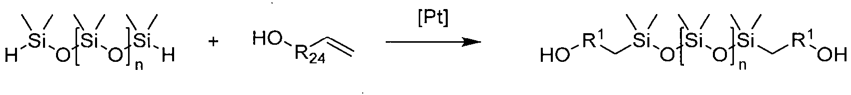

In which:
R²⁴ is a branched or linear C₁-C₁₅ alkanediyl group, preferably a branched or linear C₁-C₁₁ alkanediyl group, and more preferably a branched or linear C₁-C₅ alkanediyl group, that is interrupted by one or more O atoms and/or -C(O)O- groups and is preferably uninterrupted.

PDMS blocks bearing carboxylic acid groups (with X¹ = CH₂) can be prepared by the platinum catalyzed reaction of an unsaturated carboxylic acid with a SiH-functional PDMS:

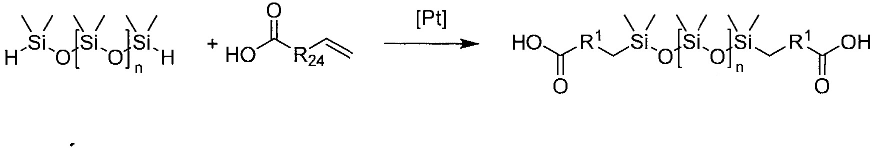

PDMS oligomers bearing a single NHR²¹, OH or COOH group (with X¹ = CH₂) can also be synthesized via hydrosilylation of SiH functional PDMS with the corresponding unsaturated compounds:

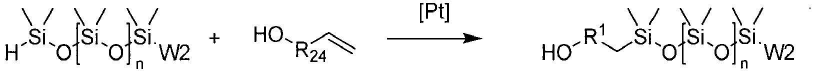

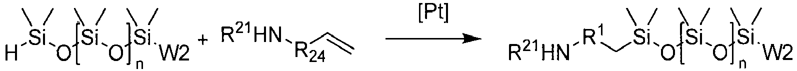

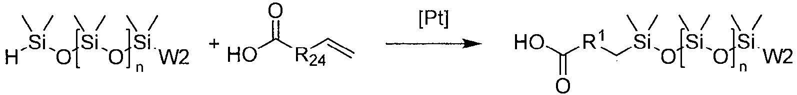

The second step of the synthesis depends on the nature of R²³.

Case 1: If R²³ is either a OH or a NHR²¹ group, the desired macro PDMS alkoxyamines can be synthesized by reacting the telechelic PDMS (Formula 7 or 8) with an alkoxyamine bearing a R²⁵ group, wherein R²⁵ is a COOH or a COOR²⁶ group, and wherein R²⁶ is a 4-nitrophenyl, a 2,3,5,6-tetrafluorphenyl or preferably a N-hydroxysuccinimide group:

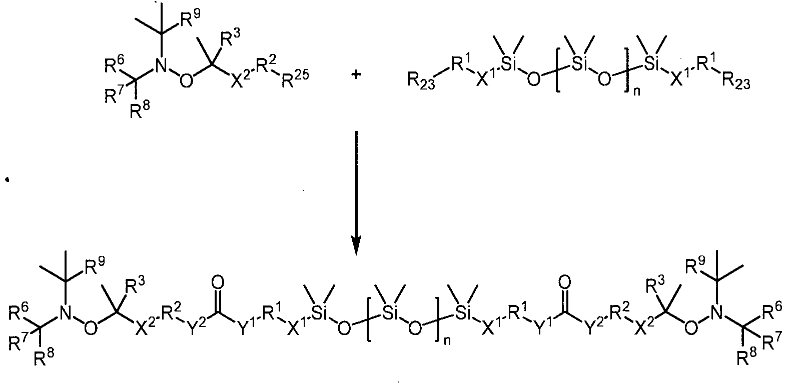

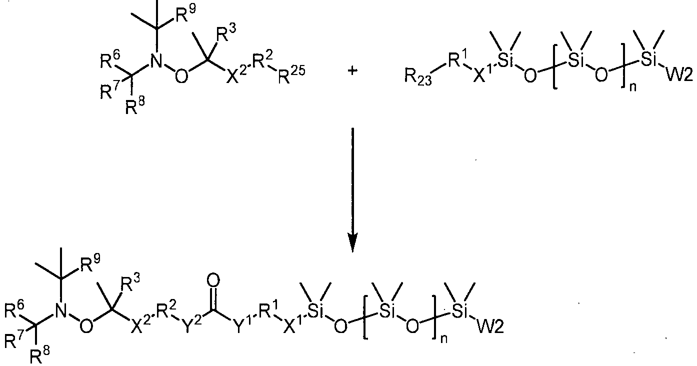

If R²³ is a OH group, Y¹ is an O atom, and if R²³ is a NHR²¹ group, Y¹ is a NR²¹ group and Y² is absent.

Case 2: If R²³ is a COOH group, the desired macro PDMS alkoxyamines can be synthesized by reacting the telechelic PDMS (Formula 7 or 8) with an alkoxyamine bearing a OH group (esterification):

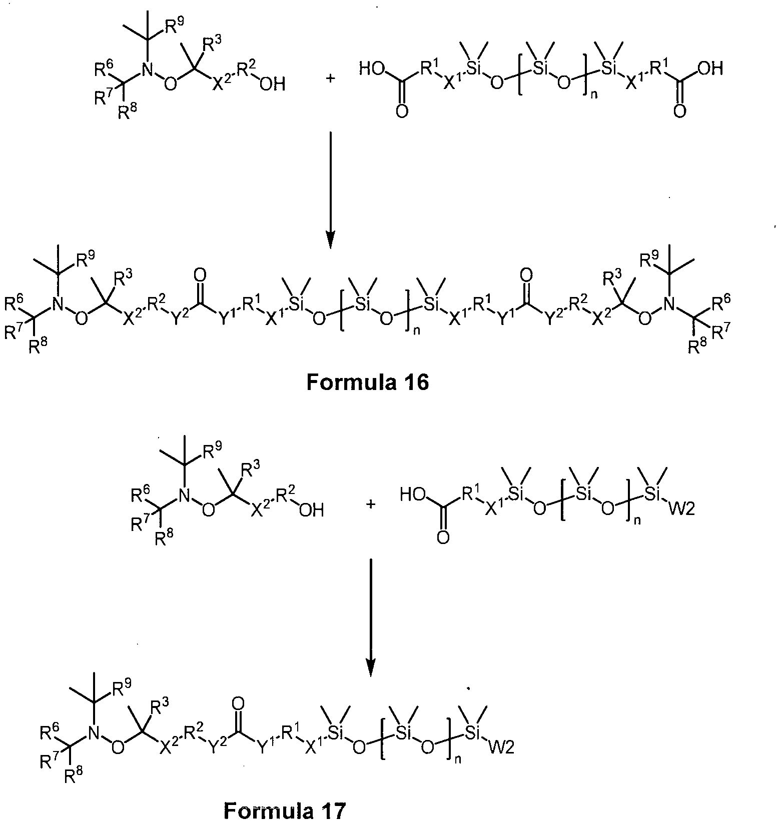

In this case, Y² is an O atom and Y¹ is absent.

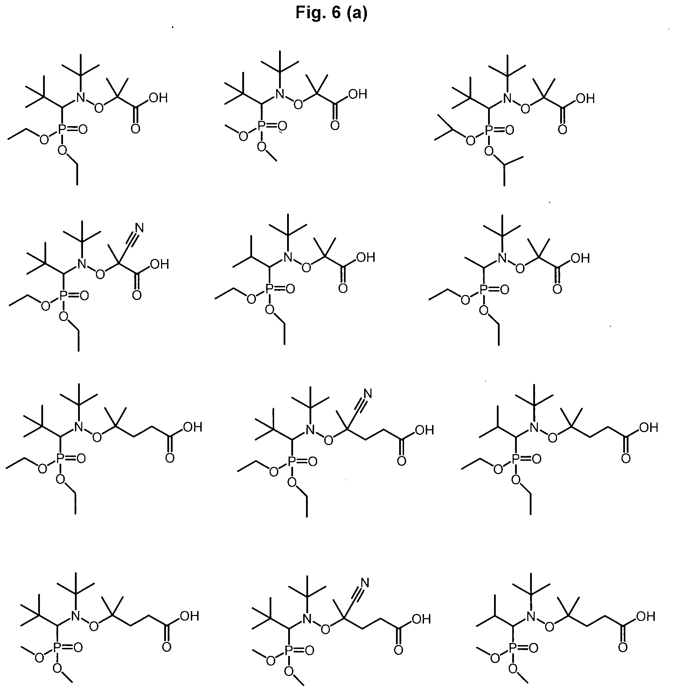

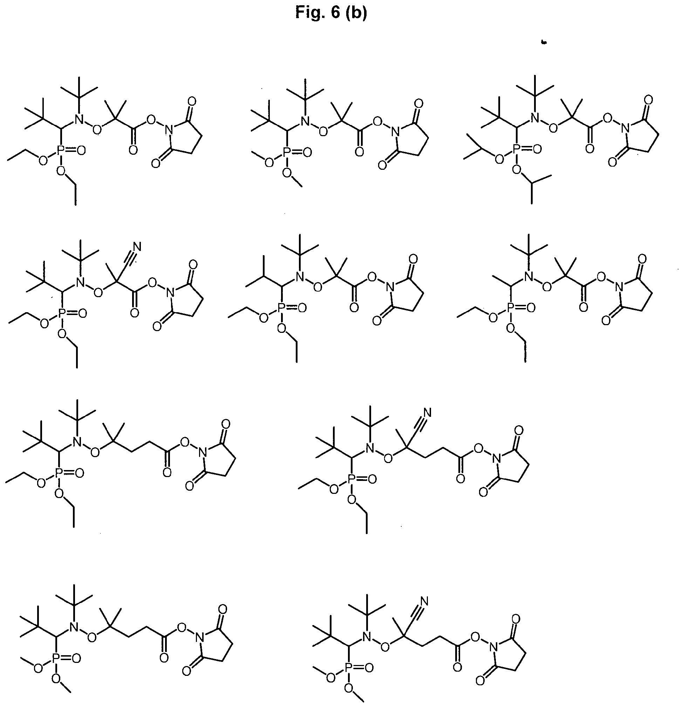

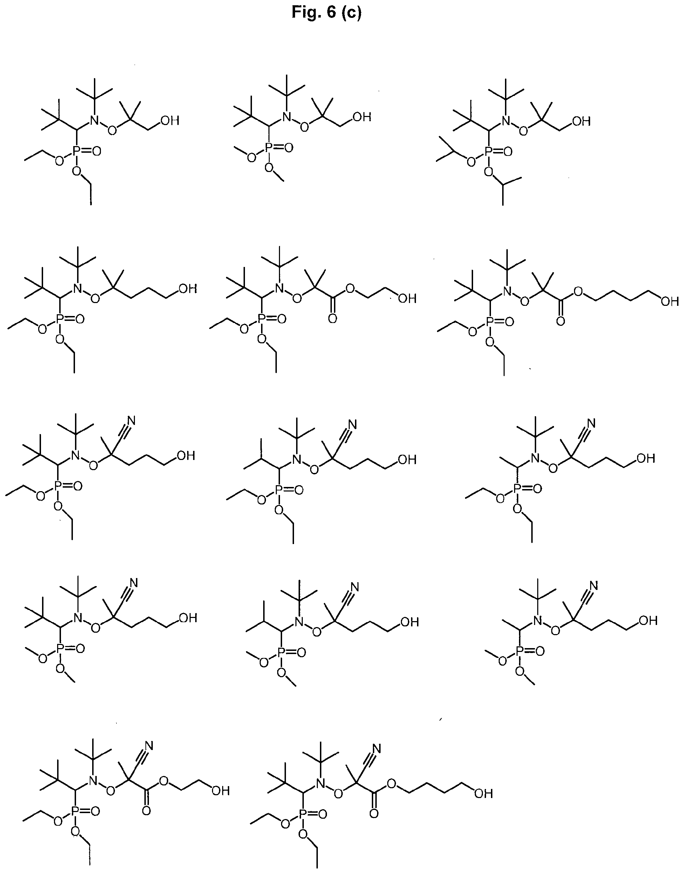

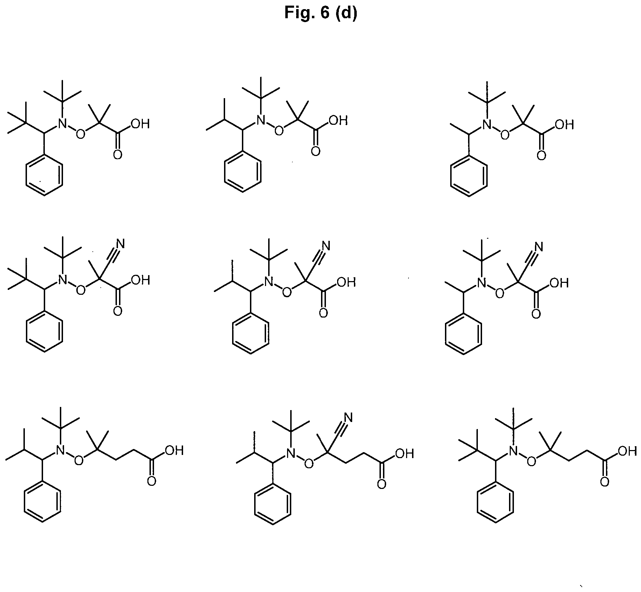

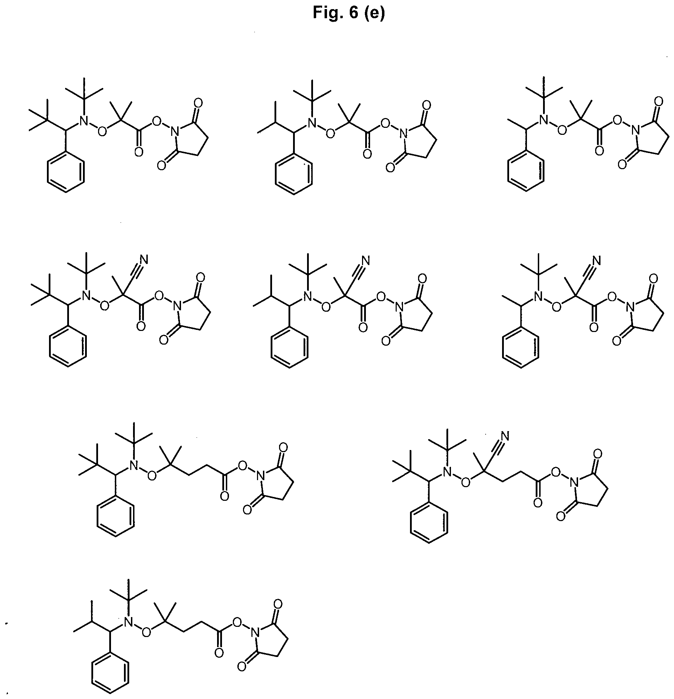

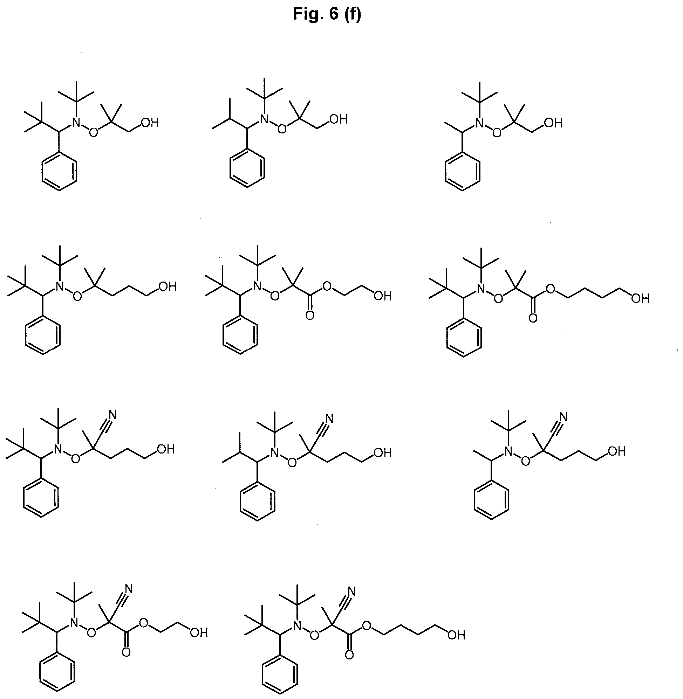

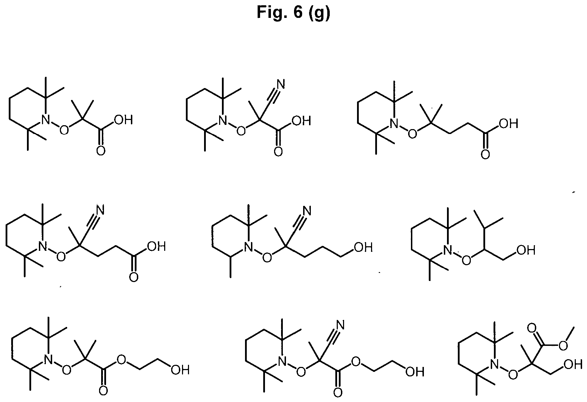

Preferred alkoxyamines for the synthesis of the PDMS alkoxyamines are shown in Figure 6. These and other suitable alkoxyamines can be synthesized in analogy to know methods, for example as described by Nicolas et al. Progress in Polymer Science 2013, 38, 79-85.

The compounds according to Formulae 16 and 17 are not known from the prior art and are also subject of the present invention.

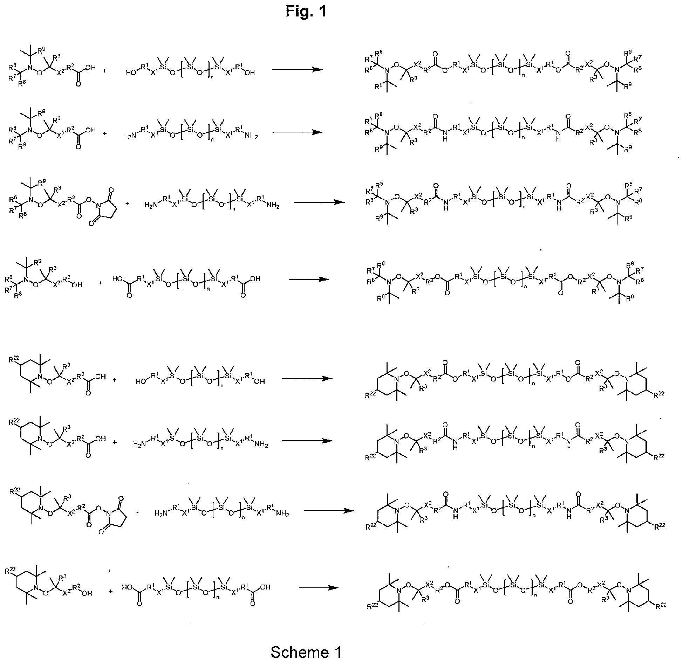

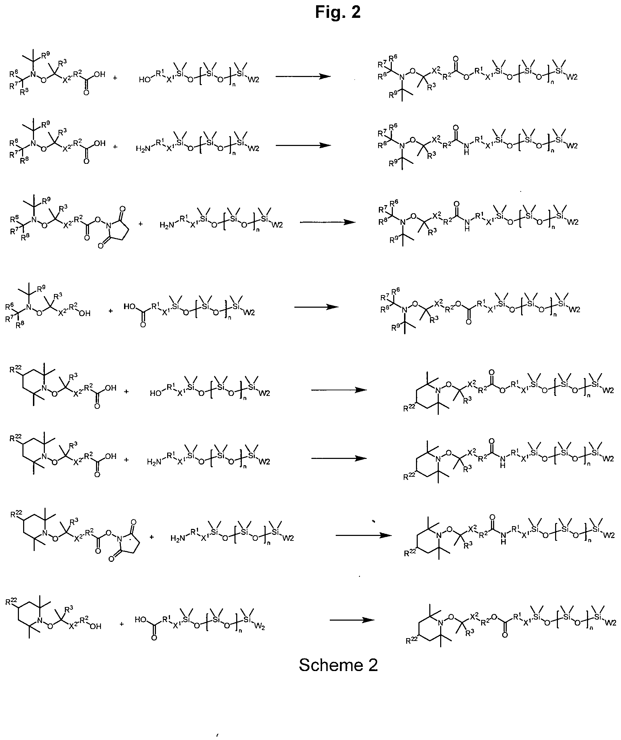

Preferred synthesis routes for the preparation of PDMS alkoxyamines according to Formula 7 are shown in Figure 1. Preferred synthesis routes for the preparation of PDMS alkoxyamines according to Formula 8 are shown in Figure 2.

In the next step, the desired block copolymers are synthesized by nitroxide-mediated radical polymerization (NMP) of one monofunctional methacrylate monomer using the PDMS alkoxyamines of Formulae 7 and 8 as macroinitiators. In order to achieve low dispersity, the NMP of methacrylate monomers is preferably carried out in the presence of at least one, preferably one, comonomer. Preferred comonomers are mono-functional acrylates and styrene. Alternatively, the block copolymers are synthesized by nitroxide-mediated radical polymerization (NMP) of a monofunctional acrylate monomer using the PDMS alkoxyamines of Formulae 7 and 8 as macroinitiators. In this case, no comonomers are required.

The synthesis of triblock copolymers is exemplified in Figure 3 and the synthesis of diblock copolymers in Figure 4. Figure 5 shows the synthesis of a particularly preferred triblock copolymer.

According to a first embodiment of the invention, the block copolymers are synthesized by NMP of a monofunctional methacrylate and a comonomer.

Preferred monofunctional methacrylates for the synthesis of the block copolymers of Formula 1 are aliphatic linear or branched C₁-C₈ alkyl methacrylates such as methyl methacrylate, ethyl methacrylate, propyl methacrylate, butyl methacrylate, pentyl methacrylate, hexyl methacrylate, isopropyl methacrylate, isobutyl methacrylate, ethylene glycol methyl ether methacrylate, diethylene glycol methyl ether methacrylate and 2-ethylhexyl methacrylate.

Further preferred monofunctional methacrylates are aromatic methacrylates such as 2-phenoxyethyl methacrylate, benzyl methacrylate, p-cumylphenoxyethylene glycol methacrylate and 2-(2-biphenyloxy)-ethyl methacrylate, and cycloaliphatic mono-functional methacrylates such as isobornyl methacrylate, furfuryl methacrylate, tetrahydrofurfuryl methacrylate, dicyclopentadienyl methacrylate and 4-tert-butylcyclohexyl methacrylate.

Additional preferred monofunctional methacrylates are methacrylates comprising, in addition to the radically polymerizable group, a functional group, preferably a hydroxyl group, such as 2-hydroxyethyl methacrylate, 2-hydroxypropyl methacrylate as well as glycol methacrylates such as ethylene glycol methyl ether methacrylate, or diethylene glycol methyl ether methacrylate.

Furthermore, monofunctional methacrylates comprising a urethane group can advantageously be used. They can be synthesized, for example, via the reaction of a hydroxyalkyl methacrylate with an isocyanate or via the reaction of an alcohol with 2-isocyanatoethyl methacrylate.

Preferred comonomers for the synthesis of the block copolymers of Formula 1 are monofunctional acrylates and in particular styrene. The NMP of a monofunctional methacrylate and a comonomer yields random copolymer blockscomprising two different repeating units.

If styrene is used, copolymer chains are obtained wherein R⁵ is a phenyl group. For the synthesis, the monofunctional methacrylate and styrene are preferably used in such amounts that the average ratio of the molar amount of styrene to the total molar amount of monofunctional methacrylate and styrene is within a range of from 0.01 to 0.5, preferably from 0.02 to 0.2, more preferably from 0.04 to 0.15.

Preferred monofunctional acrylate comonomers are aliphatic linear or branched C₁-C₈ alkyl acrylates such as methyl acrylate, ethyl acrylate, propyl acrylate, butyl acrylate, pentyl acrylate, hexyl acrylate, isopropyl acrylate, isobutyl acrylate and 2-ethylhexyl acrylate.

Further preferred monofunctional acrylate comonomers are aromatic acrylates such as 2-phenoxyethyl acrylate, benzyl acrylate, p-cumylphenoxyethylene glycol acrylate and 2-(2-biphenyloxy)-ethyl acrylate, and cycloaliphatic mono-functional methacrylates such as isobornyl acrylate, furfuryl acrylate, tetrahydrofurfuryl acrylate, dicyclopentadienyl acrylate and 4-tert-butylcyclohexyl acrylate.

Additional preferred monofunctional acrylate comonomers are acrylates comprising, in addition to the radically polymerizable group, a functional group, preferably a hydroxyl group, such as 2-hydroxyethyl acrylate, 2-hydroxypropyl acrylate as well as glycol acrylates such as ethylene glycol methyl ether acrylate, or diethylene glycol methyl ether acrylate.

If a monofunctional acrylate is used as the comonomer, copolymer chains are obtained wherein R⁵ is a -(C=O)OR¹⁵ group. The monofunctional methacrylate and the acrylate comonomer are preferably used in such amounts that the average ratio of the molar amount of monofunctional acrylate to the total molar amount of monofunctional methacrylate and acrylate comonomers is within a range of from 0.01 to 1.0, preferably from 0.02 to 1.0, more preferably from 0.04 to 1.0.

According to the second embodiment of the invention, the polymer blocks of the groups W1 and W2 are synthesized by the polymerization of styrene or one or more, preferably one, acrylate monomer using the PDMS alkoxyamine as macro initiator (R⁵ is a phenyl or -(C=O)OR¹⁵ group, q = 0). By this process, acrylate polymer blocks and preferably acrylate homopolymer blocks are obtained.

Preferred monofunctional acrylate monomers for the synthesis of the polyacrylate blocks are aliphatic linear or branched C₁-C₈ alkyl acrylates such as methyl acrylate, ethyl acrylate, propyl acrylate, butyl acrylate, pentyl acrylate, hexyl acrylate, isopropyl acrylate, isobutyl acrylate and 2-ethylhexyl acrylate.

Further preferred monofunctional acrylate monomers are aromatic acrylates such as 2-phenoxyethyl acrylate, benzyl acrylate, p-cumylphenoxyethylene glycol acrylate and 2-(2-biphenyloxy)-ethyl acrylate, and cycloaliphatic mono-functional methacrylates such as isobornyl acrylate, furfuryl acrylate, tetrahydrofurfuryl acrylate, dicyclopentadienyl acrylate and 4-tert-butylcyclohexyl acrylate.

Additional preferred monofunctional acrylate monomers are acrylates comprising, in addition to the radically polymerizable group, a functional group, preferably a hydroxyl group, such as 2-hydroxyethyl acrylate, 2-hydroxypropyl acrylate as well as glycol acrylates such as ethylene glycol methyl ether acrylate, or diethylene glycol methyl ether acrylate.

It was found that the block copolymers according to the invention significantly improve the fracture toughness of di(meth)acrylate polymer networks without significantly impairing the mechanical properties (flexural strength and modulus). The block copolymers according to the invention are compatible with a wide variety of (meth)acrylate monomers and can easily be homogeneously mixed with (meth)acrylate-based resin mixtures. Moreover, they cause only a small increase in viscosity.

Due to their high activity, comparatively small amounts of the block copolymers are needed to achieve the desired fracture toughness, with the result that it is possible to match the materials to the planned application and to set the desired fracture toughness and fracture work without problems. They are therefore particularly suited as additives for established compositions, as their addition is expected to have only minor side effects on the properties of these materials.

Furthermore, due to their alkoxyamine end-groups, the block copolymers according to the invention can be covalently incorporated to the network through a thermal post-curing step, which is advantageous with regard to the mechanical properties and long-term stability of the hardened materials.

The block copolymers of Formula 1 are particularly suitable for the preparation of **dental materials.** Preferred dental materials according to the invention have the following composition:
(a) 30 to 70 wt.%, preferably 30 to 65 wt.% and most preferably 40 to 60 wt.% of at least an aromatic, a bicyclic or a tricyclic mono(meth)acrylate,
(b) 20 to 65 wt.%, preferably 25 to 60 wt.% und most preferably 30 to 55 wt.% of at least a urethane di(meth)acrylate,
(c) 0 to 30 wt.%, preferably 0 to 20 wt.% and most preferably 0 wt.% of one or more dimethacrylate monomer without urethane groups,
(d) 1 to 15 wt.%, preferably 2 to 13 wt.% und most preferably 3 to 10 wt.% from at least one block copolymer according to Formula 1,
(e) 0.1 to 5.0 wt.%, preferably 0.2 bis 4.0 wt.% and most preferably 0.3 to 3.0 wt.% of at least one of at least one photoinitiator for the radical polymerization.

Unless otherwise stated, all percentages by weight (wt.%) herein relate to the total mass of the material.

The dental materials according to the invention preferably contain as **component (a)** at least one aromatic, bicyclic or tricyclic mono(meth)acrylate of Formula (9) 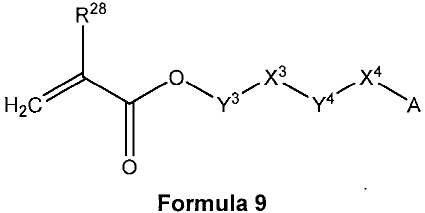 in which the variables have the following meanings:
- A: is an aromatic group with 6 to 15 carbon atoms or a bicyclic or tricyclic aliphatic group with 7 to 10 carbon atoms, wherein A can be unsubstituted or substituted by one or more C₁-C₅ alkyl groups, C₁-C₅ alkoxy groups and/or chlorine atoms;
- R²⁸: hydrogen or methyl;
- X³, X⁴: independently of each other are in each case absent or an ether, ester or urethane group, wherein X³ is absent if Y³ is absent and wherein X⁴ is absent if Y⁴ is absent;
- Y³, Y⁴: independently of each other are in each case absent or a branched or preferably linear aliphatic hydrocarbon radical with 1 to 10 carbon atoms, which can be interrupted by 1 to 3 oxygen atoms.

Herein, (meth)acrylate represents acrylate, methacrylate or a mixture thereof. Mono-functional monomers are monomers with one radically polymerizable group. Mono-functional (meth)acrylates, also referred to as mono(meth)acrylates. comprise one (meth)acrylate group. Accordingly, multifunctional monomers comprise two or more, preferably two, radically polymerizable groups. Monomers with two (meth)acrylate groups are also referred to as di(meth)acrylates. In addition to the radically polymerizable groups, monofunctional and multifunctional monomers can comprise other functional groups such as hydroxy groups or carboxy groups.

Preferred aromatic groups A are benzene, biphenyl and 2,2-diphenylpropane:

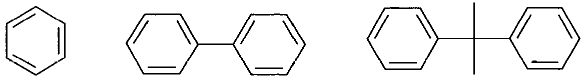

Preferred bicyclic aliphatic groups A are bicyclo[4.4.0]decane, bicyclo[4.3.0]nonane, bicyclo[2.2.2]octane and bicyclo[2.2.1]heptane:

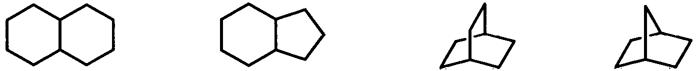

A preferred tricyclic aliphatic group A is tricyclo[5.2.1.0^{2,6}]decane:

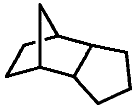

Preferred aromatic mono(meth)acrylates (a) are 2-[(benzyloxycarbonyl)-amino]-ethyl (meth)acrylate, 2-[(benzylcarbamoyl)-oxy]-ethyl (meth)acrylate, and 2-(p-cumylphe-noxy)-ethyl (meth)acrylate, More preferred aromatic mono(meth)acrylates are 2-hydroxy-3-phenoxypropyl (meth)acrylate, 2-[(benzyloxycarbonyl)amino]-ethyl (meth)-acrylate, 1-phenoxypropan-2-yl (meth)acrylate, 2-(benzyloxy)ethyl (meth)acrylate, 3-phenoxybenzyl (meth)acrylate, phenoxypropyl (meth)acrylate, 2-benzyloxyethyl (meth)acrylate, 2-benzoyloxyethyl (meth)acrylate, 2-(meth)acryloyloxybenzoic acid methyl ester, 2-phenylethyl (meth)acrylate and/or 2-(p-cumylphenoxy)ethyl (meth)-acrylate, and most preferred aromatic mono(meth)acrylates are 2-phenoxyethyl (meth)acrylate, 2-(o-biphenyloxy)ethyl (meth)acrylate.

Preferred bi- or tricyclic mono(meth)acrylates (a) are tricyclodecane (meth)acrylate, tricyclodecane methanol (meth)acrylate and isobornyl (meth)acrylate.

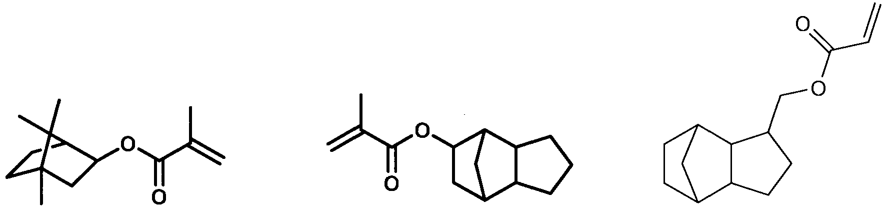

The aromatic, bicyclic or tricyclic monomethacrylates of Formula 9 used according to the invention are characterized by a good radical polymerizability. In addition, the polymers of these monomethacrylates have a comparatively low polymerization shrinkage and good mechanical properties. Because of their relatively high molar mass (150 to 350 g/mol) and their relatively non-polar structure, the mono(meth)acrylates of Formula 9 also have a low volatility and a comparatively low viscosity.

The dental materials of the present invention most preferably comprise at least one mono(meth)acrylate selected from the group consisting of 2-phenoxyethyl (meth)acrylate, 2-(o-biphenyloxy)ethyl (meth)acrylate, tricyclodecane (meth)acrylate, tricyclodecane methanol (meth)acrylate and isobornyl (meth)acrylate as component (a).

The dental materials according to the invention contain as **component (b)** at least one urethane di(meth)acrylate, preferably a urethane dimethacrylate with a molecular weight of < 1500 g/mol.

Preferred urethane dimethacrylates are monomers according to the following formulas 10-13:

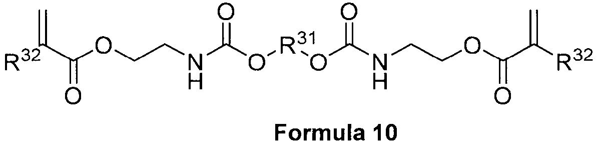

Wherein:
- R³¹: is a branched or preferably linear C₂-C₁₂ alkanediyl group, preferably a C₂-C₁₀ alkanediyl group, and more preferably a C₂-C₈ alkanediyl group, that can be interrupted by one or more O atoms and/or -C(O)O- groups; or a C₄-C₁₆ cycloaliphatic or tricyclic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and that can be interrupted by one or more O atoms and/or -C(O)O- groups; or a combination of the branched or the linear C₁-C₁₆ alkanediyl group and the C₄-C₁₆ cycloaliphatic group; or a C₇-C₃₀-alkylaryl group that can be interrupted by one or more O atoms and/or C(O)O- groups and/or can be unsubstituted or substituted by one or more substituents selected from branched or preferably linear C₁-C₂₀ alkyl groups, branched or preferably linear -O-C₁-C₁₂ alkoxy groups, ester groups -C(O)OR³³, where R³³ is a branched or preferably linear C₁-C₂₀ alkyl group and/or acyl groups -C(O)-R³⁴, where R³⁴ is a branched or preferably linear C₁-C₂₀ alkyl group;
- R³²: is H or CH₃.

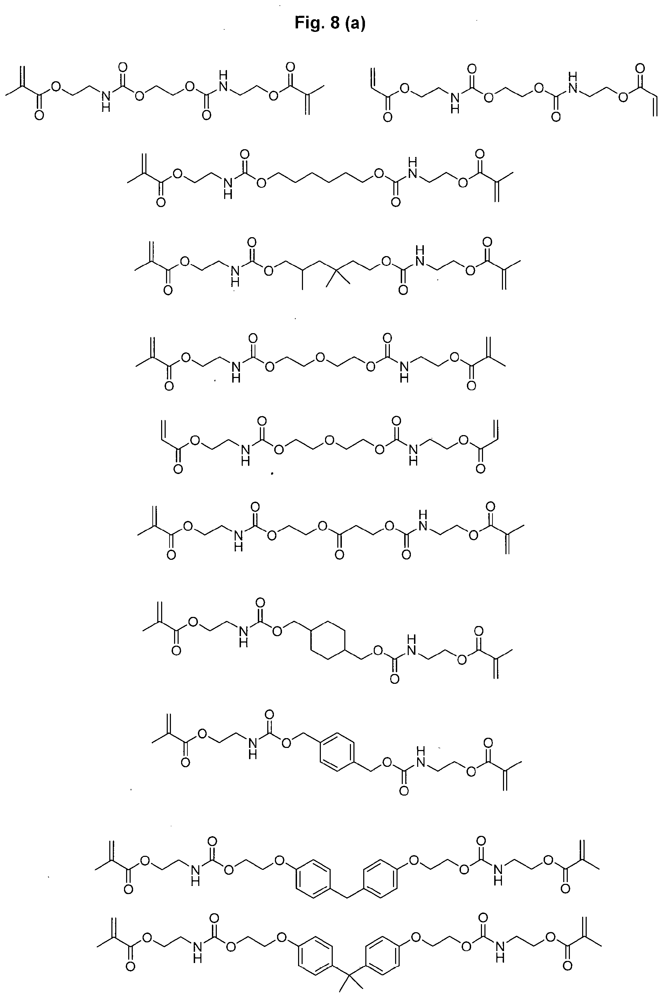

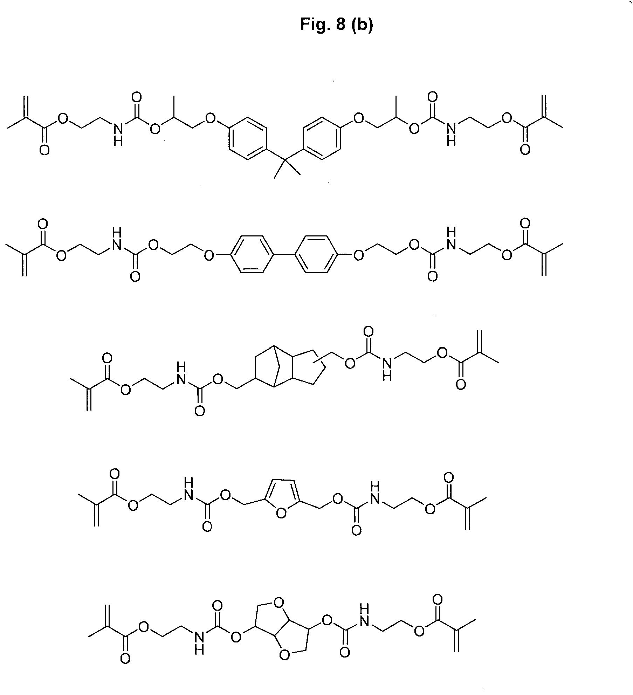

Particularly preferred urethane dimethacrylates according to Formula 10 are shown in Figure 8.

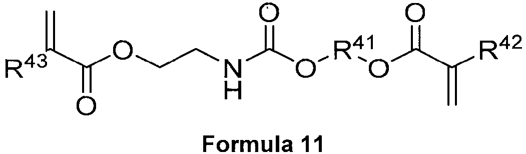

Wherein:
- R⁴¹: is a branched or preferably linear C₂-C₁₀ alkanediyl group, preferably a C₂-C₈ alkanediyl group, and more preferably a C₂-C₆ alkanediyl group, that can be interrupted by one or more O atoms and/or -C(O)O- groups;
- R⁴²: is H or CH₃;
- R⁴³: is H or CH₃.

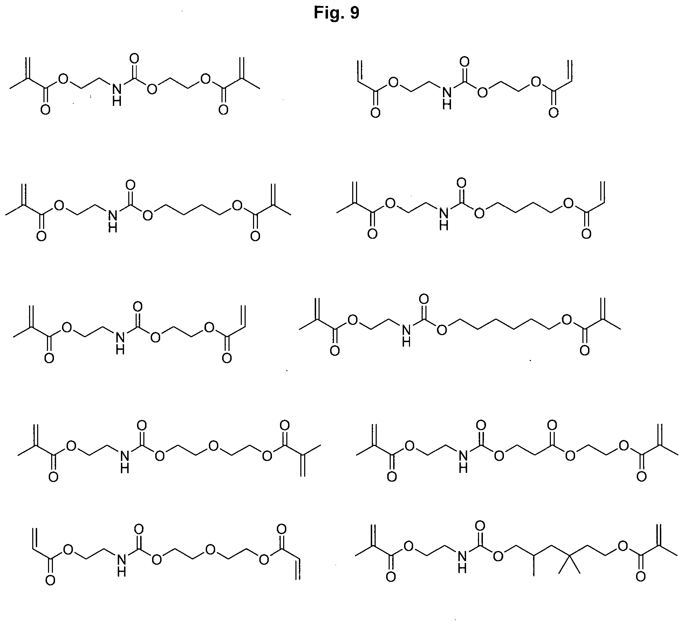

Particularly preferred urethane dimethacrylates according to Formula 11 are shown in Figure 9.

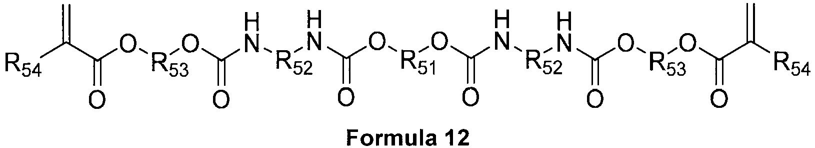

Wherein:
- R⁵¹: is a branched or preferably linear C₂-C₁₂ alkanediyl group, preferably a C₂-C₁₀ alkanediyl group, and more preferably a C₂-C₈ alkanediyl group, that can be interrupted by one or more O atoms and/or -C(O)O- groups; or a C₄-C₁₆ cycloaliphatic or tricyclic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and that can be interrupted by one or more O atoms and/or -C(O)O- groups; or a combination of the branched or the linear C₁-C₁₆ alkanediyl group and the C₄-C₁₆ cycloaliphatic group; or a C₇-C₃₀-alkylaryl group that can be interrupted by one or more O atoms and/or C(O)O- groups and/or can be unsubstituted or substituted by one or more substituents selected from branched or preferably linear C₁-C₂₀ alkyl groups, branched or preferably linear -O-C₁-C₁₂ alkoxy groups, ester groups -C(O)O-R³, where R⁸ is a branched or preferably linear C₁-C₂₀ alkyl group and/or acyl groups -C(O)-R⁴, where R⁴ is a branched or preferably linear C₁-C₂₀ alkyl group;
- R⁵²: is a linear, branched or cyclic aliphatic or an aromatic or a combined aliphatic and aromatic group containing 6 to 15 carbon atoms;
- R⁵³: is a branched or preferably linear C₂-C₁₀ alkanediyl group, preferably a C₂-C₁₈ alkanediyl group, and more preferably a C₂-C₆ alkanediyl group, that can be interrupted by one or more O atoms and/or -C(O)O- groups;
- R⁵⁴: is H or preferably CH₃.

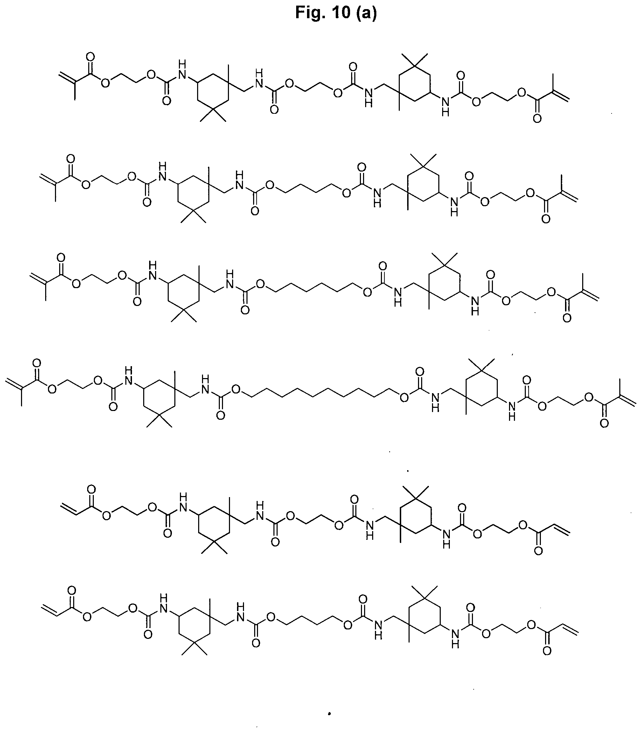

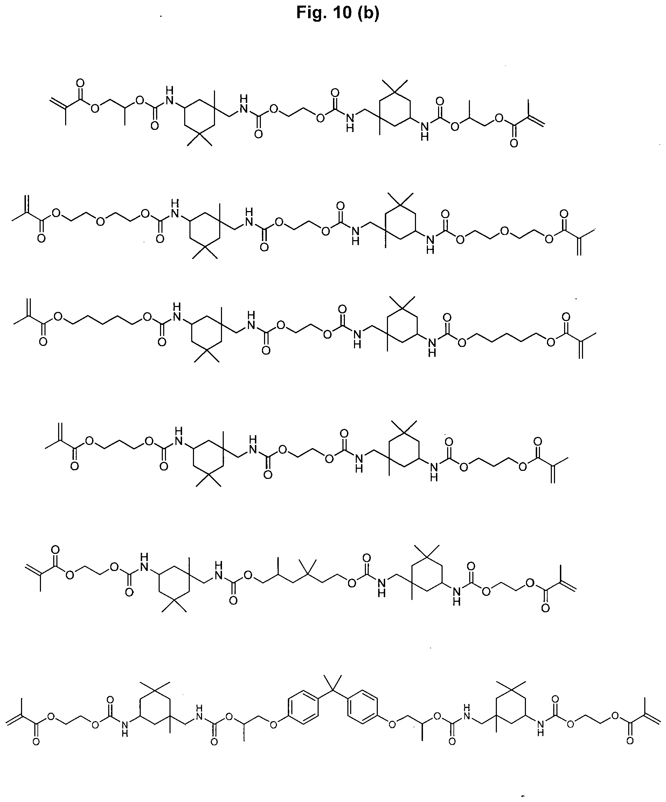

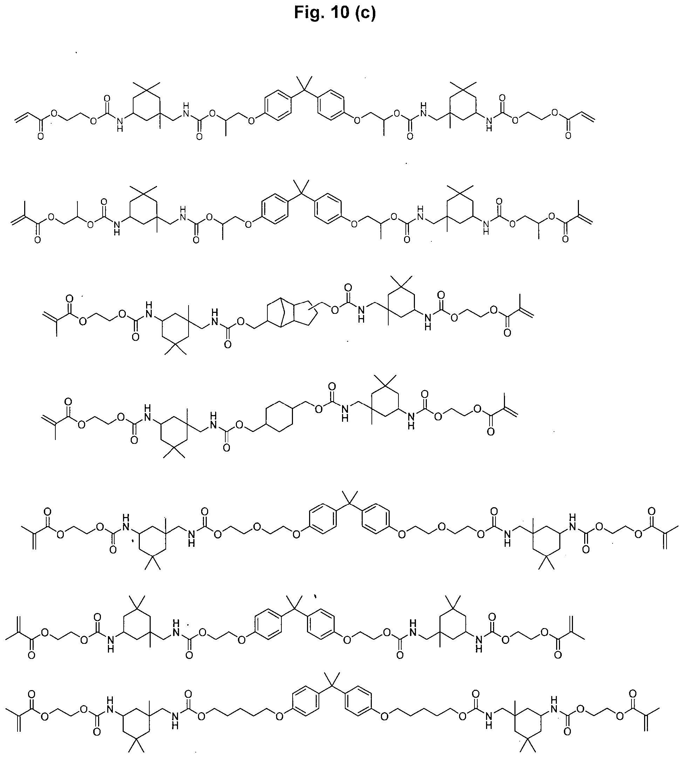

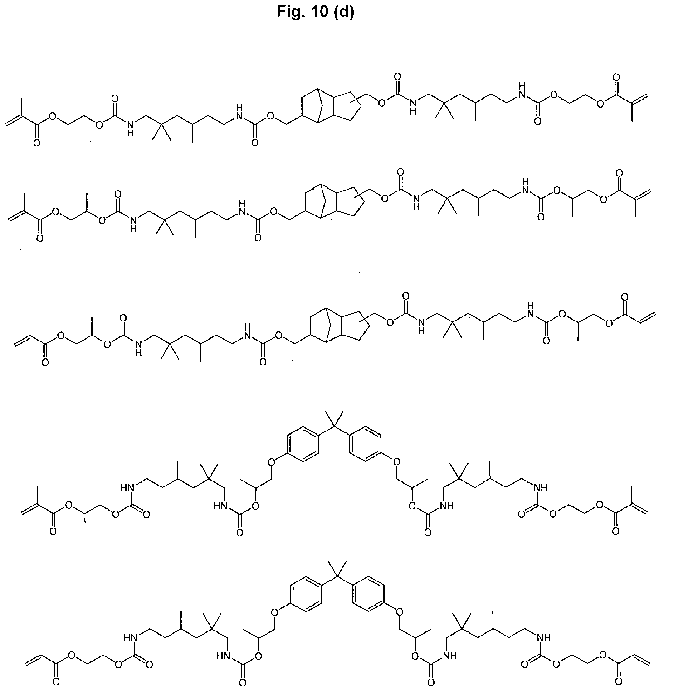

Particularly preferred urethane dimethacrylates according to Formula 12 are shown in Figure 10.

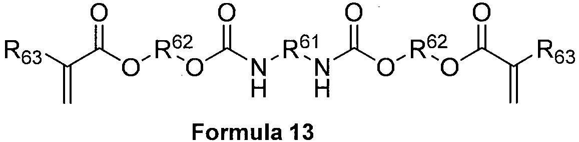

Wherein:
- R⁶¹: is a linear, branched or cyclic aliphatic or an aromatic or a combined aliphatic and aromatic group containing 6 to 15 carbon atoms;
- R⁶²: is a branched or preferably linear C₂-C₁₀ alkanediyl group, preferably a C₂-C₈ alkanediyl group, and more preferably a C₂-C₆ alkanediyl group, that can be interrupted by one or more O atoms and/or -C(O)O- groups;
- R⁶³: H or CH₃.

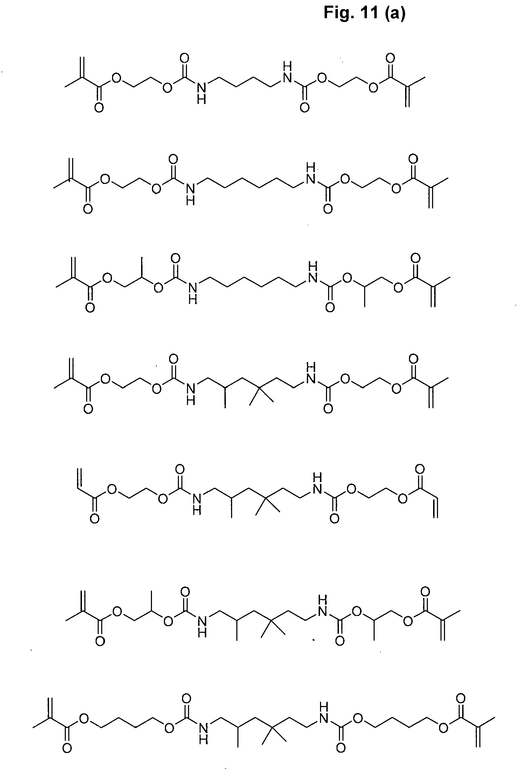

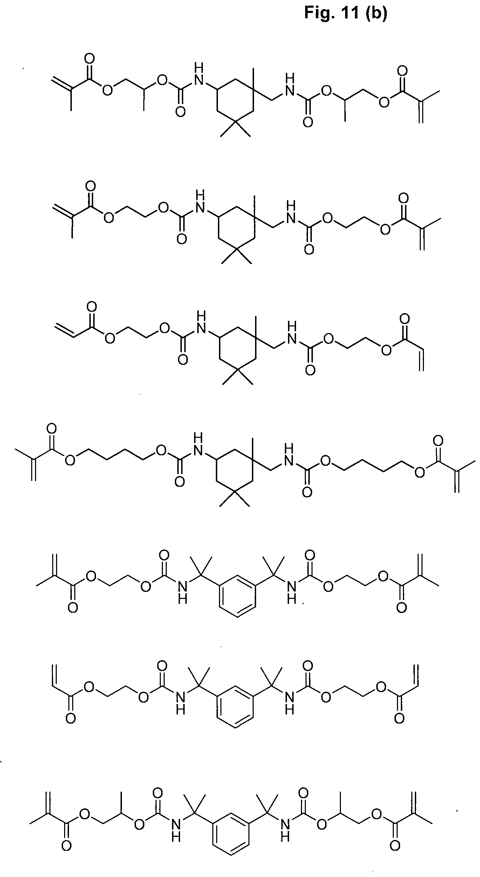

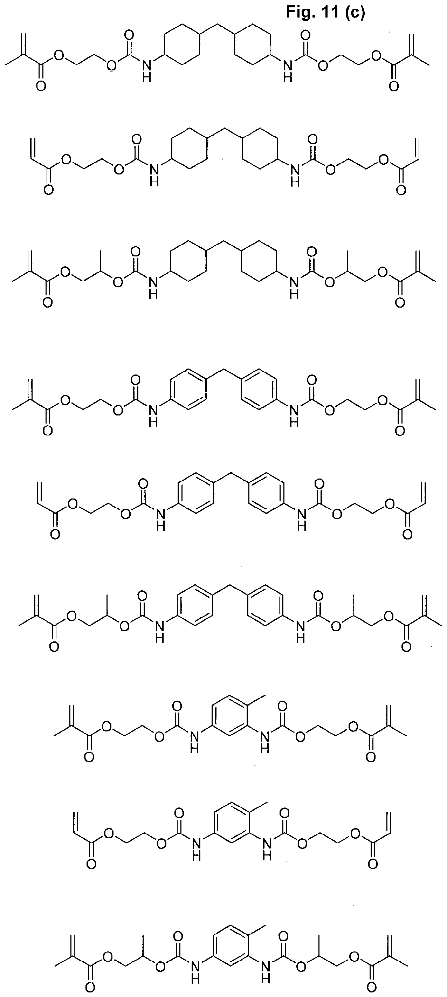

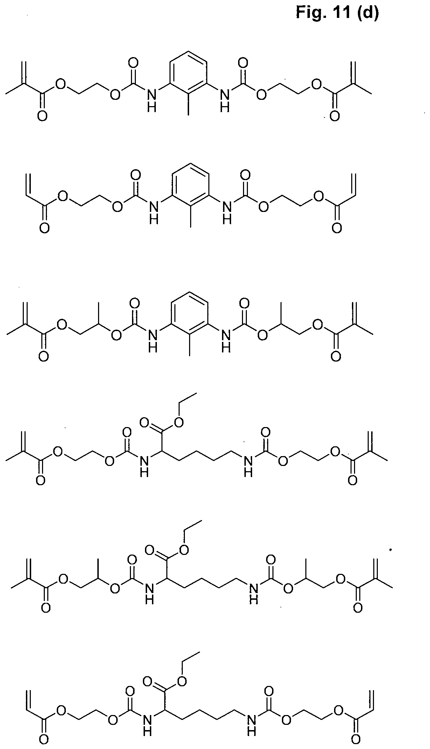

Particularly preferred urethane dimethacrylates according to the Formula 13 are shown in Figure 11.

The dental materials of the present invention most preferably comprise at least one urethane dimethacrylate according to Formula 12 as component (b).

Urethane dimethacrylates according to Formula 10 can preferably be obtained by the reaction of two mols of 2-isocyanatoethyl (meth)acrylate with one mol of a diol according to the following reaction scheme:

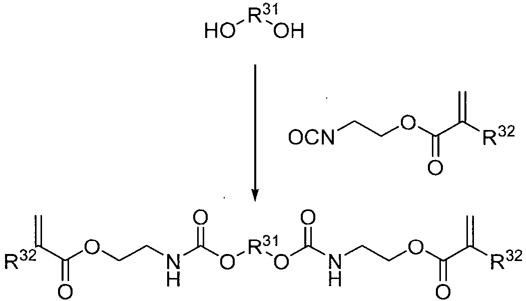

Urethane dimethacrylates according to Formula 11 can preferably be obtained by the reaction of one mol of 2-isocyanatoethyl (meth)acrylate with one mol of a hydroxy(meth)acrylate derivative:

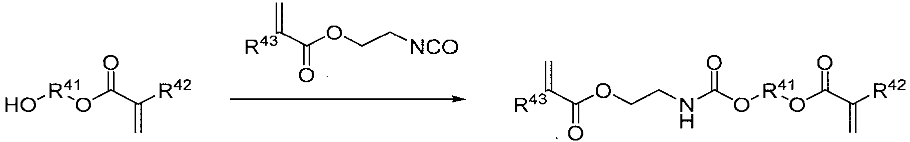

Urethane dimethacrylates according to Formula 12 can preferably be obtained by reaction of one mol of a diol with 2 mol of a diisocyanate, followed by the end-capping with two mols of a hydroxy(meth)acrylate:

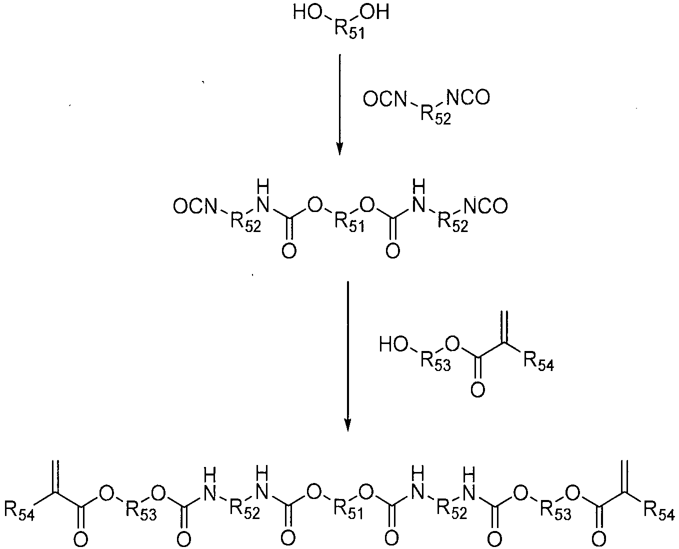

Urethane dimethacrylates according to Formula 13 can preferably be obtained by the reaction of one mol of a diisocyanate with two mols of a hydroxy(meth)acrylate derivative:

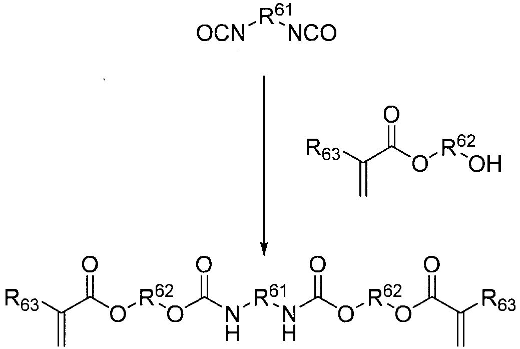

Preferred diisocyanates for the synthesis of urethane dimethacrylates according to Formulae 10 and 11 are toluene 2,4-diisocyanate (2,4-TDI), toluene 2,6-diisocyanate (2,6-TDI) and mixtures of isomers (TDI); 4,4'-diphenyl methane diisocyanate (4,4'-MDI), 2,4'-diphenyl methane diisocyanate (2,4'-MDI), 2,2'-diphenyl methane diisocyanate (2,2'-MDI) and mixtures of isomers (MDI); 1,6-diisocyanatohexane (HDI); 5-iso-cyabato-1-(isocyanatomethy)-1,3,3-trimethylcyclohexane (IPDI); 1,3-bis(1-isocyanato-1-methylethy) benzene (TMXDI); 1,1'-methylebis(4-isocyanato-cyclohexane) (HMDI); 4,4'-o-dibenzyl diisocyanate (DBDI); 1,6-diisocyanato-2,4,4-trimethylhexane, 1,6-diisocyanato-2,2,4-trimethylhexane and mixtures of isomers (TMDI); 1,5-diisocyanatonaphthalene (NDI); 1,3-phenylene diisocyanate; 1,4-phenylene diisocyanate (PPDI); 1,3-Xylylene diisocyanate (m-XDI); 4,4'-Diisocyanato-3,3'-dimethyl-1,1'-biphenyl (TODI); 1,4-diisocyanatobutane; ethyl (2S)-2,6-diisocyanatohexanoate (LDI); 1,3-bis(isocyanatomethyl)cyclohexane (hydrogenated m-XDI); 1,1'-oxybis[4-isocyanatobenzene], and more preferred diisocyanates are HDI, IPDI, TMXDI, HMDI and TMDI. The most preferred diisocyanates are IPDI and TMDI.

The dental materials according to the invention can additionally contain one or more **dimethacrylate monomers (c)** that do not bear a urethane group.

Preferred dimethacrylates without urethane groups are bisphenol A dimethacrylate (bis-GMA, an addition product of methacrylic acid and bisphenol A diglycidyl ether), ethoxylated or propoxylated bisphenol A dimethacrylate, such as e.g. the bisphenol A dimethacrylate SR-348c (Sartomer) with 3 ethoxy groups, bis(methacryloyloxymethyl)-tricyclo-[5.2.1.0^{2,6}]decane (DCP), di-, tri- or tetraethylene glycol dimethacrylate, glycerol dimethacrylate, acylated glycerol dimethacrylate and 1,4-butanediol dimethacrylate.

The dental materials according to the invention contain as **component (e)** at least one initiator for the radical polymerization, preferably a photoinitiator.

Preferred photoinitiators are benzophenone, benzoin and their. derivatives as well as α-diketones and their derivatives, such as 9,10-phenanthrenequinone, 1-phenyl-propane-1,2-dione, diacetyl or 4,4'-dichlorobenzil. Camphorquinone (CQ) and 2,2-dimethoxy-2-phenyl-acetophenone are particularly preferably used, and α-diketones in combination with amines as reducing agent, such as e.g. 4-(dimethylamino)benzoic acid ester (EDMAB), N,N-dimethylaminoethyl methacrylate, N,N-dimethyl-sym.-xylidine or triethanolamine, are quite particularly preferably used.

Further preferred photoinitiators according to the invention are oxime esters such as 1-[4-(phenylthio)phenyl]-1,2-octanedione 2-(O-benzoyloxime) (Irgacure OXE-01) or O-acetyl-1-[6-(2-methylbenzoyl)-9-ethyl-9H-carbazol-3-yl]ethanone oxime (Irgacure OXE-02).

Further preferred photoinitiators according to the invention are monomolecular photoinitiators, also called Norrish Typ I photoinitiators, such as monoacyltrialkyl-, diacyldialkyl- and tetraacylgermanium as well as tetraacylstannanes. Benzoyltrimethylgermanium, dibenzoyldiethylgermanium, bis(4-methoxybenzoyl)diethylgermanium, tetrakis-(2-methylbenzoyl)germane or tetrakis(mesitoyl)stannane are particularly preferred. More preferred photoinitiators are acyl- or bisacylphosphine oxides, in particular Irgacure TPO (2,4,6-trimethylbenzoyldiphenylphosphine oxide), ethyl (2,4,6-trimethylbenzoyl) phenylphosphinate (TPO-L), Irgacure 819 (bis(2,4,6-trimethylbenzoyl)phenylphosphine oxide), (2,4-dimethoxy-6-methylbenzoyl)diphenylphosphine oxide and (2,4,6-trimethoxybenzoyl)diphenylphosphine oxide.

For post-curing, it is advantageous to use two photoinitiators which differ in their absorption ranges, such as Irgacure TPO and camphorquinone/4-(dimethylamino)benzoic acid ester.

The dental materials according to the invention can additionally also contain thermal initiators, e.g. azo compounds, such as 2,2'-azobis(isobutyronitrile) (AIBN) or azobis-(4-cyanovaleric acid), or peroxides, such as dibenzoyl peroxide, dilauroyl peroxide, *tert*-butyl peroctoate, *tert*-butyl perbenzoate or di-(*tert*-butyl) peroxide.

The thermal initiators are preferably used in a total quantity of from 0.1 to 5.0 wt.%, particularly preferably 0.2 to 4.0 wt.% and quite particularly preferably 0.3 to 3.0 wt.%.

To improve the mechanical properties, the dental materials according to the invention can be strengthened with **inorganic particulate fillers (f)**.

Preferred inorganic fillers are oxides, such as SiO₂, ZrO₂ and TiO₂ or mixed oxides of SiO₂, ZrO₂, ZnO and/or TiO₂, nanoparticulate or microfine fillers, such as fumed silica or precipitated silica, glass powders, such as quartz, glass ceramic, borosilicate or radiopaque glass powders, preferably barium or strontium aluminium silicate glasses, and radiopaque fillers, such as ytterbium trifluoride, tantalum(V) oxide, barium sulfate or mixed oxides of SiO₂ with ytterbium(III) oxide or tantalum(V) oxide. The dental materials according to the invention can furthermore contain fibrous fillers, nanofibres, whiskers or mixtures thereof. According to a preferred embodiment, the materials according to the invention do not contain fluoroaluminosilicate glasses, calcium aluminium silicate glasses or other fillers which react with organic acids in the sense of an acid-base reaction.

Preferably, the oxides have a particle size of from 0.010 to 15 µm, the nanoparticulate or microfine fillers have a particle size of from 10 to 300 nm, the glass powders have a particle size of from 0.01 to 15 µm , preferably of from 0.2 to 1.5 µm, and the radiopaque fillers have a particle size of from 0.2 to 5 µm.

Particularly preferred fillers are mixed oxides of SiO₂ and ZrO₂, with a particle size of from 10 to 300 nm, glass powders with a particle size of from 0.2 to 1.5 µm, in particular radiopaque glass powders of e.g. barium or strontium aluminium silicate glasses, and radiopaque fillers with a particle size of from 0.2 to 5 µm, in particular ytterbium trifluoride and/or mixed oxides of SiO₂ with ytterbium(III) oxide.

To improve the bond between the filler particles and the crosslinked polymerization matrix, SiO₂-based fillers can be surface-modified with methacrylate-functionalized silanes. A preferred example of such silanes is 3-methacryloyloxypropyltrimethoxysilane. For the surface modification of non-silicate fillers such as ZrO₂ or TiO₂, functionalized acidic phosphates, such as e.g. 10-methacryloyloxydecyl dihydrogen phosphate can also be used.

Further preferred fillers are particulate waxes, in particular carnauba wax, preferably with a particle size of from 1 to 10 µm, non-crosslinked or partially crosslinked polymethyl methacrylate (PMMA) particles, preferably with a particle size of from 500 nm to 10 µm, as well as polyamide-12 particles, preferably with a particle size of from 5 to 10 µm.

Moreover, the dental materials according to the invention can contain a so-called prepolymer filler or isofiller, i.e. a ground composite which preferably has a broad particle-size distribution, e.g. with particle sizes of from 0.05 to 20 µm, in particular approximately 0.1 to approximately 10 µm. The prepolymer filler or isofiller is preferably surface modified, in particular silanized.

Preferred fillers according to the invention are silanized glass fillers with a particle size of 0.2 to 1.5 µm, and more preferred fillers are silanized silica nanoparticles

The total filler content preferably lies in a range of from 0 to 20 wt.%, particularly preferably of from 0 to 10 wt.%.

Unless otherwise stated, all particle sizes herein are weight-average particle sizes, wherein the particle-size determination in the range of from 0.1 µm to 10 µm is effected by means of static light scattering, preferably using an LA-960 static laser scattering particle size analyser (Horiba, Japan). Here, a laser diode with a wavelength of 655 nm and an LED with a wavelength of 405 nm are used as light sources. The use of two light sources with different wavelengths makes it possible to measure the entire particle-size distribution of a sample in only one measurement pass, wherein the measurement is carried out as a wet measurement. For this, a 0.1 to 0.5% aqueous dispersion of the filler is prepared and the scattered light thereof is measured in a flow cell. The scattered-light analysis for calculating particle size and particle-size distribution is effected in accordance with the Mie theory according to DIN/ISO 13320.

Particle sizes smaller than 0.1 µm are preferably determined by means of dynamic light scattering (DLS). The measurement of the particle size in the range of from 5 nm to 0.1 µm is preferably effected by dynamic light scattering (DLS) of aqueous particle dispersions, preferably using a Malvern Zetasizer Nano ZS (Malvern Instruments, Malvern UK) with an He-Ne laser with a wavelength of 633 nm, at a scattering angle of 90° at 25°C.

The light scattering decreases as the particle size decreases. Particle sizes smaller than 0.1 µm can also be determined by means of SEM or TEM spectroscopy. The transmission electron microscopy (TEM) is preferably carried out using a Philips CM30 TEM at an accelerating voltage of 300 kV. For the preparation of the samples, drops of the particle dispersion are applied to a 50 Å thick copper grid (mesh size 300), which is coated with carbon, and then the solvent is evaporated.

The dental materials according to the invention can furthermore contain one or more **UV absorbers (g)**. The UV absorber serves to reduce the penetration depth of the light, and thus the polymerization depth, during the light-induced curing of the composition according to the invention. This proves to be advantageous in particular in the case of stereolithographic applications as only thin layers are to be cured in stereolithography. The use of a UV absorber can improve the precision in stereolithographic processes.

UV absorbers based on benzotriazole, benzophenone or triazines are preferred. Particularly preferred UV absorbers are 2,2'-methylenebis[6-(2H-benzotriazol-2-yl)-4-(1,1,3,3-tetramethylbutyl)phenol], 2,2',4,4'-tetrahydroxybenzophenone, 2-tert-butyl-6-(5-chloro-2H-benzotriazol-2-yl)-4-methylphenol (bumetrizole), 2,2'-benzene-1,4-diylbis(4H-3, 1-benzoxazin-4-one), 2-(4,6-bis-(2,4-dimethylphenyl)-1,3,5-triazin-2-yl)-5-(octyloxy)-phenol, 2-(2-hydroxy-5-methylphenyl)benzotriazole, 2-(2-hydroxyphenyl)-benzotriazole, 2-(2H-benzotriazol-2-yl)-6-dodecyl-4-methylphenol, 2-(2'-hydroxy-3',5'-di-t-butylphenyl)-5-chlorobenzotriazole, 2,2'-dihydroxy-4-methoxybenzophenone and 2,2'-dihydroxy-4,4'-dimethoxybenzophenone. So-called Hindered Amine Light Stabilizers such as bis(1,2,2,6,6-pentamethyl-4-piperidyl) sebacate, methyl-1,2,2,6,6-pentamethyl-4-piperidyl sebacate, bis(1-octyloxy-2,2,6,6-tetramethyl-4-piperidyl) sebacate and bis(1,2,2,6,6-pentamethyl-4-piperidyl)-[[3,5-bis(1,1-dimethylethyl)-4-hydroxyphenyl]methyl]butyl malonate are further preferred. Quite particularly preferred UV absorbers are bumetrizole and 2,2',4,4'-tetrahydroxybenzophenone.

The UV absorber preferably has an absorption maximum which corresponds to the wavelength of the light used for the curing. UV absorbers with an absorption maximum in the range of from 320 to 500 nm and preferably 380 to 480 nm are advantageous, wherein UV absorbers with an absorption maximum below 400 nm are particularly preferred.

UV absorbers are optionally used in a quantity of from preferably 0 to 1.0 wt.%, particularly preferably 0.01 to 0.5 wt.%. Bumetrizole is preferably used in a quantity of from 0.01 to 0.2 wt.%, particularly preferably 0.02 to 0.15 wt.%, and 2,2',4,4'-tetrahydroxybenzophenone in a quantity of from 0.01 to 0.07 wt.%. All data relate to the total weight of the material. Dental materials which do not contain a UV absorber are preferred.

The dental materials according to the invention can also contain one or more **optical brighteners (h)**. Optical brighteners which absorb light in the UV range, i.e. light with a wavelength below 400 nm, are preferred according to the invention. Through the addition of an optical brightener, the penetration depth of the light, and thus the curing depth, can be reduced and the precision in stereolithographic processes can thus be increased. Optical brighteners which are capable of re-emitting light absorbed in the UV range as light with a wavelength of from 400 to 450 nm are particularly preferred. Such optical brighteners increase the reactivity of the materials because, due to their fluorescence, they emit the absorbed short-wave light as longer-wave blue light and thus provide additional luminous power for the photoinitiation. Optical brighteners preferred according to the invention are 2,5-bis(5-tert-butyl-benzoxazol-2-yl)thiophene and fluorescent agents in the form of terephthalic acid derivatives, such as e.g. 2,5-dihydroxyterephthalic acid diethyl ester or diethyl-2,5-dihydroxyterephthalate.

The optical brightener or optical brighteners are optionally used in a quantity of from preferably 0 to 0.1 wt.%, particularly preferably 0.001 to 0.05 wt.% and quite particularly preferably 0.002 to 0.02 wt.%, in each case relative to the total weight of the material. Dental materials which do not contain an optical brightener are preferred.

Optical brighteners can be used in combination with UV absorbers. In this case, it is preferred that the weight ratio of UV absorber to optical brightener lies in a range of from 2:1 to 50:1, particularly preferably 2:1 to 30:1 and quite particularly preferably 2:1 to 5:1 or 10:1 to 25:1. Combinations which contain 2,2',4,4'-tetrahydroxybenzophenone or bumetrizole as UV absorber and 2,5-bis(5-tert-butyl-benzoxazol-2-yl)thiophene as optical brightener are preferred. The combination of 2,2',4,4'-tetrahydroxybenzophenone and 2,5-bis(5-tert-butyl-benzoxazol-2-yl)thiophene in a weight ratio of from 2:1 to 10:1, preferably 2:1 to 5:1, or the combination of bumetrizole and 2,5-bis(5-tert-butyl-benzoxazol-2-yl)thiophene in a weight ratio of from 5:1 to 30:1, preferably 10:1 to 20:1, is quite particularly preferred.

The dental materials according to the invention can additionally contain **further additives (i),** above all stabilizers, colorants, plasticizers, thixotropic additives, microbiocidal active ingredients and/or foaming agents.

The dental materials according to the invention preferably contain one or more **stabilizers.** These are free-radical-scavenging substances for preventing a premature polyreaction. The stabilizers are also called polymerization inhibitors. The inhibitors or stabilizers improve the storage stability of the materials.

Preferred inhibitors are phenols, such as hydroquinone monomethyl ether (MEHQ) or 2,6-di-tert-butyl-4-methylphenol (BHT). Phenols are preferably used in a concentration of from 0.001 to 0.50 wt.%. Further preferred inhibitors are phenothiazine, the 2,2-diphenyl-1-picrylhydrazyl (DPPH) radical, the galvinoxyl radical, the triphenylmethyl radical and the 2,2,6,6-tetramethylpiperidine-1-oxyl (TEMPO) radical. These inhibitors are preferably used in a quantity of from 0.001 to 0.02 wt.%. A polymerization does not take place until these additives have been used up. The quantities relate in each case to the total mass of the material. A mixture of inhibitors which contains at least one phenol and at least one of the further initiators is preferably used.

In addition, the dental materials according to the invention can also contain **colorants,** preferably in a concentration of from 0.0001 to 0.5 wt.%. The colorants are primarily used for aesthetic purposes. Colorants preferred according to the invention are organic dyes and pigments, in particular azo dyes, carbonyl dyes, cyanine dyes, azomethines and methines, phthalocyanines and dioxazines. Dyes which are soluble in the materials according to the invention, in particular azo dyes, are particularly preferred. Moreover, inorganic and in particular organic pigments which can be dispersed well in the dental materials according to the invention are suitable as colorant. Preferred inorganic pigments are metal oxides or hydroxides, such as e.g. titanium dioxide or ZnO as white pigments, iron oxide (Fe₂O₃) as red pigment or iron oxyhydroxide (FeOOH) as yellow pigment. Preferred organic pigments are azo pigments, such as e.g. monoazo yellow and orange pigments, diazo pigments or β-naphthol pigments, and non-azo or polycyclic pigments, such as e.g. phthalocyanine, quinacridone, perylene and flavanthrone pigments. Azo pigments and non-azo pigments are particularly preferred.

The dental materials according to the invention can furthermore contain one or more **thixotropic additives.** These additives bring about a thickening of the materials and can thus, for example, prevent the fillers from sedimenting. In particular, filler-containing materials therefore preferably contain at least one thixotropic additive. Preferred thixotropic additives are OH group-containing polymers, such as e.g. cellulose derivatives, and inorganic substances, such as e.g. layer silicates. In order not to increase the viscosity of the materials too much, the dental materials according to the invention preferably contain only 0 to 3.0 wt.%, particularly preferably 0 to 2.0 wt.% and quite particularly preferably 0.1 to 2.0 wt.% thixotropic additive, relative to the total weight of the material.

Certain fillers, such as e.g. highly dispersed SiO₂, i.e. SiO₂ with a small primary particle size (< 20 nm) and a large surface area (> 100 m²), likewise have a thixotropic effect. Such fillers can replace thixotropic additives.

The rheological properties of the dental materials according to the invention are matched to the desired intended application. Materials for stereolithographic processing are preferably adjusted such that their viscosity lies in the range of from 50 mPa·s to 100 Pa·s, preferably 100 mPa·s to 10 Pa.s, particularly preferably 100 mPa·s to 5 Pa s. The viscosity is determined at 25°C using a cone-plate viscometer (shear rate 100/s). The dental materials according to the invention particularly preferably have a viscosity < 10 Pa·s and quite particularly preferably < 5 Pa·s at 25°C. The viscosity is preferably determined using an Anton Paar MCR 302-type viscometer with a CP25-2 cone-plate measuring system and a measuring gap of 53 µm in rotation at a shear rate of 100/s. Because of the low viscosity, the dental materials according to the invention are particularly suitable for being processed using additive manufacturing processes, such as e.g. 3D printing or stereolithography. The processing temperature preferably lies in a range of from 10 to 70°C, particularly preferably 20 to 30°C.

Thus, the present invention includes dental materials comprising, in addition to components (a) to (e):
(f) 0 to 20 wt.%, preferably of from 0 to 10 wt.% and more preferably 0 wt.% of one or more fillers; and/or
(g) 0 to 1.0 wt.%, preferably of from 0.01 to 0.5 wt.% and more preferably of from 0.01 to 0.2 wt.% of one or more UV absorbers, preferably bumetrizole, 2,2',4,4'-tetrahydroxybenzophenone; and/or
(h) 0 to 0.1 wt.%, preferably of from 0.001 to 0.05 wt.% and more preferably of from 0.002 to 0.02 wt.% of one or more optical brighteners, 2,5-bis(5-tert-butyl-benzoxazol-2-yl)thiophene, fluorescent agents in the form of terephthalic acid derivatives.

According to a preferred embodiment, the dental materials of the present invention comprise:
(a) 30 to 70 wt.%, preferably 30 to 65 wt.% and more preferably 40 to 60 wt.% of at least one monofunctional (meth)acrylate selected from 2-phenoxyethyl (meth)-acrylate, 2-(o-biphenyloxy)ethyl (meth)acrylate, tricyclodecane (meth)acrylate, tricyclodecane methanol (meth)acrylate or isobornyl (meth)acrylate;
(b) 20 to 65 wt.%, preferably 25 to 60 wt.% and more preferably 30 to 55 wt.% of a urethane di(meth)acrylate according to Formula 12 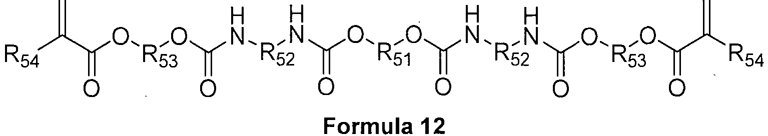 wherein:
   - R⁵¹: is a branched or preferably linear C₂-C₁₂ alkanediyl group, preferably a C₂-C₁₀ alkanediyl group, and more preferably a C₂-C₈ alkanediyl group, that can be interrupted by one or more O atoms and/or -C(O)O- groups; or a C₄-C₁₆ cycloaliphatic or tricyclic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and that can be interrupted by one or more O atoms and/or -C(O)O- groups; or a combination of the branched or the linear C₁-C₁₆ alkanediyl group and the C₄-C₁₆ cycloaliphatic group; or a C₇-C₃₀-alkylaryl group that can be interrupted by one or more O atoms and/or C(O)O- groups and/or can be unsubstituted or substituted by one or more substituents selected from branched or preferably linear C₁-C₂₀ alkyl groups, branched or preferably linear -O-C₁-C₁₂ alkoxy groups, ester groups -C(O)O-R³, where R⁸ is a branched or preferably linear C₁-C₂₀ alkyl group and/or acyl groups -C(O)-R⁴, where R⁴ is a branched or preferably linear C₁-C₂₀ alkyl group;
   - R⁵²: is a linear, branched or cyclic aliphatic or an aromatic or a combined aliphatic and aromatic group containing 6 to 15 carbon atoms;
   - R⁵³: is a branched or preferably linear C₂-C₁₀ alkanediyl group, preferably a C₂-C₁₈ alkanediyl group, and more preferably a C₂-C₆ alkanediyl group, that can be interrupted by one or more O atoms and/or -C(O)O- groups;
   - R⁵⁴: is CH₃;
(d) 1 to 15 wt.%, preferably 2 to 13 wt.% und more preferably 3 to 10 wt.% of the at least one block copolymer according to Formula 1,
(e) 0.1 to 5.0 wt.%, preferably 0.2 to 4.0 wt.% and more preferably 0.3 to 3.0 wt.% of at least one photoinitiator.

According to a second preferred embodiment, the dental materials of the present invention comprise:
(a) 30 to 70 wt.%, preferably 30 to 65 wt.% and more preferably 40 to 60 wt.% of at least one monofunctional (meth)acrylate selected from 2-phenoxyethyl (meth)-acrylate, 2-(o-biphenyloxy)ethyl (meth)acrylate, tricyclodecane (meth)acrylate, tricyclodecane methanol (meth)acrylate or isobornyl (meth)acrylate;
(b) 20 to 65 wt.%, preferably 25 to 60 wt.% and more preferably 30 to 55 wt.% of a urethane di(meth)acrylate according to Formula 12; 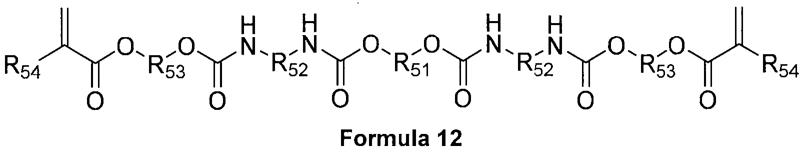 wherein:
   - R⁵¹: is a branched or preferably linear C₂-C₁₂ alkanediyl group, preferably a C₂-C₁₀ alkanediyl group, and more preferably a C₂-C₈ alkanediyl group, that can be interrupted by one or more O atoms and/or -C(O)O- groups; or a C₄-C₁₆ cycloaliphatic or tricyclic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and that can be interrupted by one or more O atoms and/or -C(O)O- groups; or a combination of the branched or the linear C₁-C₁₆ alkanediyl group and the C₄-C₁₆ cycloaliphatic group; or a C₇-C₃₀-alkylaryl group that can be interrupted by one or more O atoms and/or C(O)O- groups and/or can be unsubstituted or substituted by one or more substituents selected from branched or preferably linear C₁-C₂₀ alkyl groups, branched or preferably linear -O-C₁-C₁₂ alkoxy groups, ester groups -C(O)O-R³, where R⁸ is a branched or preferably linear C₁-C₂₀ alkyl group and/or acyl groups -C(O)-R⁴, where R⁴ is a branched or preferably linear C₁-C₂₀ alkyl group;
   - R⁵²: is a linear, branched or cyclic aliphatic or an aromatic or a combined aliphatic and aromatic group containing 6 to 15 carbon atoms;
   - R⁵³: is a branched or preferably linear C₂-C₁₀ alkanediyl group, preferably a C₂-C₁₈ alkanediyl group, and more preferably a C₂-C₆ alkanediyl group, that can be interrupted by one or more O atoms and/or -C(O)O- groups;
   - R⁵⁴: is CH₃;
(d) 1 to 15 wt.%, preferably 2 to 13 wt.% und more preferably 3 to 10 wt.% of triblock copolymer according to Formula 14 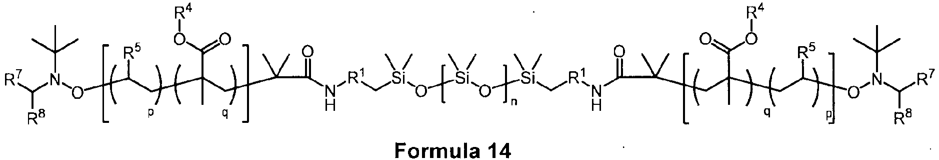 wherein:
   - R¹: is a linear C₁-C₄ alkanediyl group;
   - R⁴: is a linear C₁-C₈ alkyl group, that is uninterrupted or interrupted by 1 to 2, O atoms;
   - R⁵: is a phenyl group or a -(C=O)OR¹⁵ group in which
   - R¹⁵: is a branched or preferably linear C₁-C₈ alkyl group or a C₄-C₁₆ cycloaliphatic group that is unsubstituted or substituted by one or more, preferably 1 to 5, and more preferably 1 to 3, branched or preferably linear C₁-C₆ alkyl groups, or a C₄-C₁₀ aryl or heteroaryl group or a C₅-C₂₀-alkylaryl or C₅-C₂₀-alkylheteroaryl group
   - R⁷: is a -CH(CH₃)₂ group or a -C(CH₃)₃ group;
   - R⁸: is a P(O)(OR²⁰)₂ group in which R²⁰ is linear C₁-C₄ alkyl group or a phenyl group;
   if R⁵ is a phenyl group, p and q have the following meanings:
   - p: is a value of from 1 to 10;
   - q: is a value of from 10 to 140;
   - p + q: is > 5;
   - p/(p+q): is a value of from 0.04 to 0.15;
   if R⁵ is a -(C=O)OR¹⁵ group, p and q have the following meanings:
   - p: is a value of from 6 to 140;
   - q: is a value of from 0 to 140;
   - p + q: is > 5;
   - p/(p+q): is a value of from 0.04 to 1.0;
   - n: is a number of from 15 to 150;
(e) 0.1 to 5.0 wt.%, preferably 0.2 to 4.0 wt.% and more preferably 0.3 to 3.0 wt.% of at least one photoinitiator.

According to a third preferred embodiment, the dental materials of the present invention comprise:
(a) 30 to 70 wt.%, preferably 30 to 65 wt.% and more preferably 40 to 60 wt.% of at least one monofunctional (meth)acrylate selected from 2-phenoxyethyl (meth)acrylate, 2-(o-biphenyloxy)ethyl (meth)acrylate, tricyclodecane (meth)acrylate, tricyclodecane methanol (meth)acrylate or isobornyl (meth)acrylate;
(b) 20 to 65 wt.%, preferably 25 to 60 wt.% and more preferably 30 to 55 wt.% of a urethane di(meth)acrylate according to Formula 12 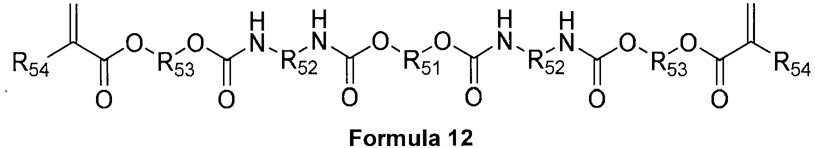 wherein:
   - R⁵¹: is a branched or preferably linear C₂-C₁₂ alkanediyl group, preferably a C₂-C₁₀ alkanediyl group, and more preferably a C₂-C₈ alkanediyl group, that can be interrupted by one or more O atoms and/or -C(O)O- groups;
   or
   a C₄-C₁₆ cycloaliphatic or tricyclic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and that can be interrupted by one or more O atoms and/or -C(O)O- groups;
   or
   a combination of the branched or the linear C₁-C₁₆ alkanediyl group and the C₄-C₁₆ cycloaliphatic group;
   or
   a C₇-C₃₀-alkylaryl group that can be interrupted by one or more O atoms and/or C(O)O- groups and/or can be unsubstituted or substituted by one or more substituents selected from branched or preferably linear C₁-C₂₀ alkyl groups, branched or preferably linear -O-C₁-C₁₂ alkoxy groups, ester groups -C(O)O-R³, where R⁸ is a branched or preferably linear C₁-C₂₀ alkyl group and/or acyl groups -C(O)-R⁴, where R⁴ is a branched or preferably linear C₁-C₂₀ alkyl group;
   - R⁵²: is a linear, branched or cyclic aliphatic or an aromatic or a combined aliphatic and aromatic group containing 6 to 15 carbon atoms;
   - R⁵³: is a C₂-C₆ alkanediyl group;
   - R⁵⁴: is CH₃;
(d) 1 to 15 wt.%, preferably 2 to 13 wt.% und more preferably 3 to 10 wt.% of triblock copolymer according to Formula 15 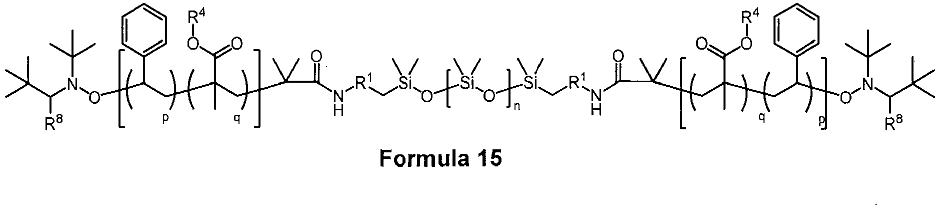 wherein:
   - R¹: is a linear C₁-C₄ alkanediyl group;
   - R⁴: is a linear C₁-C₈ alkyl group, that is uninterrupted or interrupted by 1 to 2 O atoms;
   - R⁸: is a P(O)(OR²⁰)₂ group in which R²⁰ is linear C₁-C₄ alkyl group;
   - p: is a value of from 1 to 10;
   - q: is a value of from 10 to 140;
   - p + q: is > 5;
   - p/(p+q): is a value of from 0.04 to 0.15;
   - n: is a number between 15 and 150;
(e) 0.1 to 5.0 wt.%, preferably 0.2 to 4.0 wt.% and more preferably 0.3 to 3.0 wt.% of a phosphine oxide as photoinitiator, preferably Irgacure TPO (2,4,6-trimethylbenzoyldiphenylphosphine oxide), ethyl (2,4,6-trimethylbenzoyl) phenylphosphinate (TPO-L), Irgacure 819 (bis(2,4,6-trimethylbenzoyl)phenylphosphine oxide, (2,4-dimethoxy-6-methylbenzoyl)diphenylphosphine oxide and/or (2,4,6-trimethoxybenzoyl)diphenylphosphine oxide.

A further aspect of the present invention is a process for the production of dental shaped parts, in particular for the production of the above-named dental shaped parts, in which a composition according to the invention is cured with the aid of light in order to give the dental shaped part. The production or repair of dental shaped parts is preferably effected extraorally, particularly preferably by an additive process, quite particularly preferably by 3D printing or a lithography-based process, such as e.g. stereolithography.

The stereolithographic production of shaped parts is preferably performed by creating a virtual image of the tooth situation by direct or indirect digitization of the tooth to be restored or of the teeth to be restored on a computer, then constructing a model of the dental restoration or prosthesis on the computer on the basis of this image and subsequently producing this model by additive stereolithographic manufacturing.

Once the virtual model of the dental workpiece to be produced has been created, the composition according to the invention is polymerized by selective light irradiation. The dental restoration or prosthesis is preferably constructed in layers by polymerizing a plurality of thin layers with the desired cross section one after another. After the layered construction of the restoration or prosthesis, excess residual resin is preferably removed. This can be effected by suitable mechanical processes (e.g. centrifuging or sandblasting) or by treatment with a suitable solvent, such as e.g. an alcohol, such as ethanol or isopropanol, a ketone, such as e.g. acetone, or an ester, such as e.g. ethyl acetate. A post-tempering is then preferably performed by heating or particularly preferably by irradiation of the workpiece with light of a suitable wavelength, such as e.g. irradiation with light with an intensity of e.g. 160 mW/cm² at 405 nm. When two photoinitiators are used, irradiation with two different wavelengths is advantageous. The workpiece is preferably heated to a temperature above 50°C at the same time or in a subsequent step. The mechanical properties can be improved through the photochemical and/or thermal post-tempering.

It was found that materials according to the present invention exhibit both excellent mechanical properties and high fracture toughness. Preferred materials according to the invention achieve a flexural strength of more than 60 MPa and a flexural modulus of more than 2000 MPa measured under humid conditions, i.e,. the mechanical properties are determined following ISO4049:2019, but the measurements are performed with the test specimen immersed in a water bath at 37°C to simulate the conditions in the mouth. The fracture toughness Kₘₐₓ is above 1.2 MPa·m^{1/2}. The fracture toughness is measured in analogy to ISO20795-1:2013 as described in the examples.

The invention is explained in more detail in the following with reference to figure and examples.
**Figure 1** shows synthesis routes for the production of PDMS oligomers with alkoxyamine groups by reacting PDMS oligomers with activated alkoxyamines. The PDMS alkoxyamines are suitable as initiators for the synthesis of triblock copolymers of Formula 1.
**Figure 2** shows synthesis routes for the production of PDMS alkoxyamines for the synthesis of diblock copolymers of Formula 1.

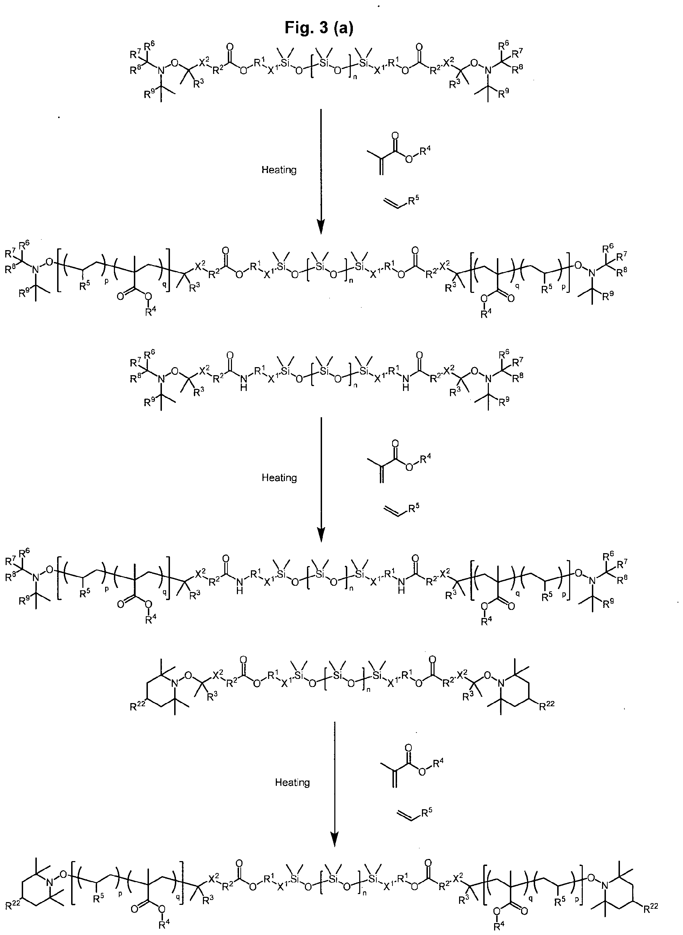

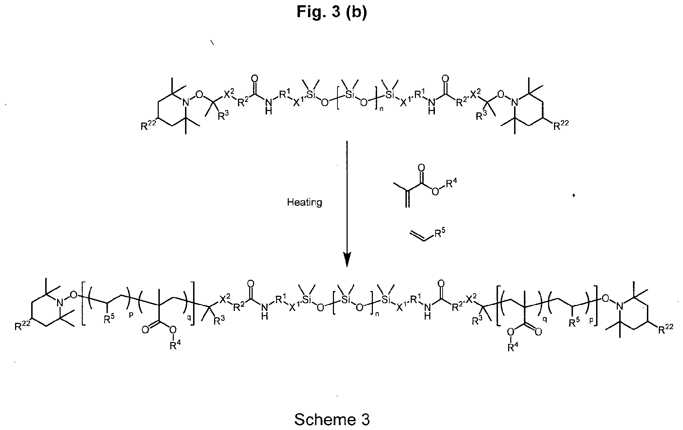

**Figure 3** illustrates the synthesis of triblock copolymers by nitroxide-mediated radical polymerization (NMP) of a monofunctional methacrylate and a comonomer using PDMS alkoxyamines as macroinitiators.

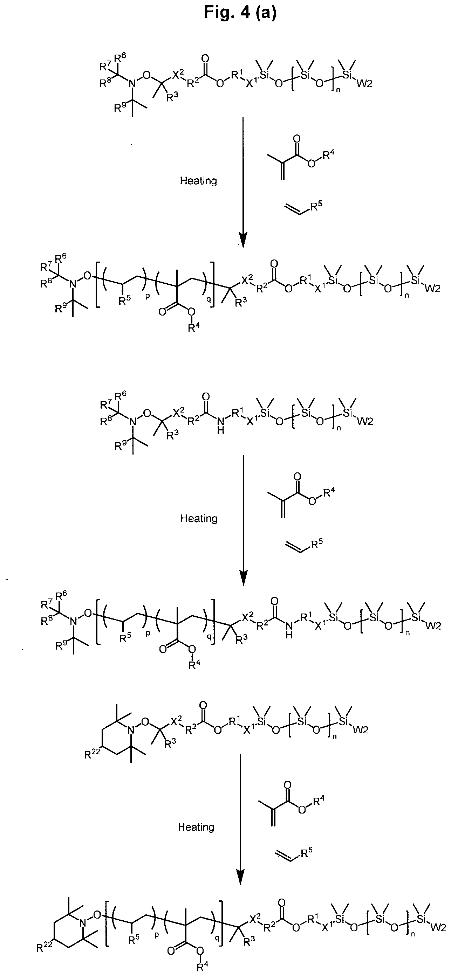

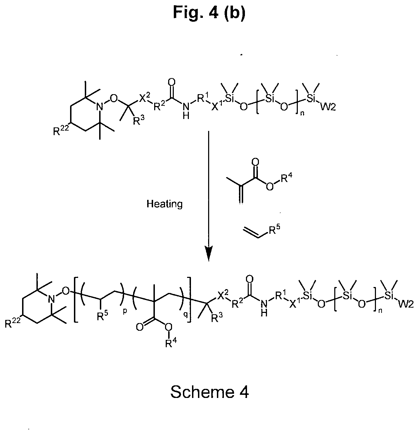

**Figure 4** illustrates the synthesis of diblock copolymers by nitroxide-mediated radical polymerization (NMP) of a monofunctional methacrylate and a comonomer using PDMS alkoxyamines as macroinitiators.

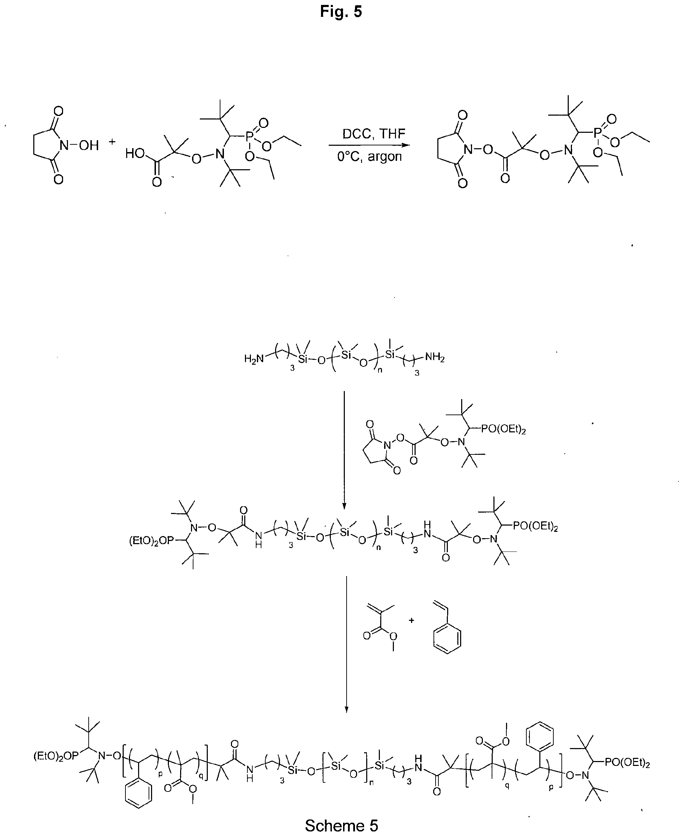

**Figure 5** shows the synthesis of a particularly preferred triblock copolymer of the present invention.
**Figure 6** shows preferred alkoxyamines for the synthesis of the PDMS alkoxyamines used in the synthesis routes shown in Figures 1 and 2.
In **Figure 7****,** preferred block copolymers according to the invention are shown.
**Figure 8** shows preferred urethane dimethacrylates according to Formula 10.
**Figure 9** shows preferred urethane dimethacrylates according to Formula 11.
**Figure 10** shows preferred urethane dimethacrylates according to Formula 12.
**Figure11** shows preferred urethane dimethacrylates according to Formula 13.

### Examples

### Example 1:

### Synthesis of the urethane dimethacrylate DMA 1

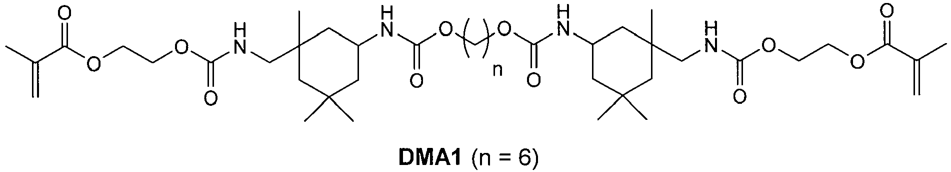

A mixture of 1 equivalent 1,6- hexanediol, 2 equivalents isophorone diisocyanate (IPDI) and 475 ppm dibutyltin dilaurate (relative to diol) was heated to 100°C. The diol dissolved completely, and the mixture heated up to 120°C. After the exothermicity had subsided, the mixture was stirred at 100°C bath temperature for 1 h. After cooling down to 90°C, 2.05 equivalents 2-hydroxyethyl methacrylate (HEMA, stabilized with 30 ppm (relative to 100% product) BHT) were added dropwise and temperature rose to 130°C. After the exothermicity had subsided again, stirring was continued for a further 60 min at 90°C. The completeness of the reaction was checked by IR and NMR spectroscopy. DMA1 was obtained as colourless, highly viscous resin.

¹H NMR (400 MHz, CDCl₃): δ (ppm) = 0.85-0.97, 1.01-1.06, 1.19-1.38 and 1.59-1.75 [4 m, 38H, (CH₂)₄, CH_{2, cycl.}, CH_{3, cycl.}), 1.95 (s, 6H, CH₃, methacryl), 2.91-2.94 and 3.20-3.33 (2 m, 4H, NCH₂), 3.65-3.88 [m, 2H, NCH], 4.01-4.06 (m, 4H, OCH₂(CH₂)₈), 4.28-4.36 (m, 8H, O(CH₂)₂O), 4.57-4.97 (m, 4H, NH), 5.60 and 6.14 (2 s, each 2H, =CH₂). IR (diamond ATR): v (cm⁻¹) = 3332 (br, NH), 2952 (m, br C-H), 1694 (vs, C=O), 1638 (m, C=C), 1527 (s, NH), 1455 (m, CH₂, CH₃), 1366 (w, CH₃), 1301 (m, CH₃), 1236 (C-N), 1167 and 1042 (s, COC), 845 (m, =CH), 815 and 774 [m, (CH₂)₈].

### Example 2:

### Synthesis of 2-((tert-butyl(1-(diethoxyphosphoryl)-2,2-dimethylpropyl) amino)oxy)-2-methylpropanoic acid activated with N-hydroxysucciimide(MAMASG1-NHS)

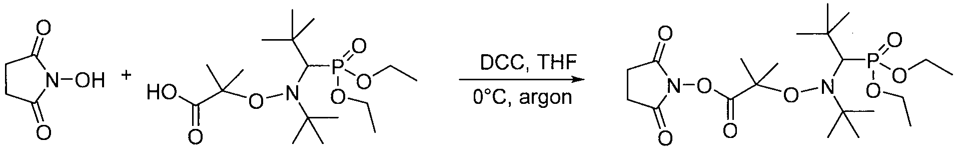

2-((*tert*-Butyl(1-(diethoxyphosphoryl)-2,2-dimethylpropyl) amino)oxy)-2-methylpropanoic acid (MAMA-SG1) (15.00 g, 39.39 mmol), tetrahydrofuran (THF, 70.0 mL), and N-hydroxysuccinimide (6.63 g, 57.65 mmol) were subsequently added in a round bottom flask under argon. The solution was stirred at room temperature for 15 min and then chilled down at 0°C using an ice bath. Then, a solution of *N*,*N*'-dicyclohexylcarbodiimide (DCC, 9.15 g, 44.42 mmol) in THF (20.0 mL) was slowly added to the reaction bottom flask. After finishing the addition, the solution was continuously stirred under argon at 0°C. After 3 h, the white solid was removed by filtration and the filtrate was concentrated under vacuum to one-third volume and placed at 20°C for 2 h and filtered to remove the residue white solid. After filtration, the filtrate was concentrated under reduced pressure and precipitation in pentane. The solid product was obtained by filtration and washed with water and n-pentane and dried under vacuum at room temperature. The final product was isolated as a white solid (12.59 g). Yield: 67%.

¹H-NMR (CDCl₃, 400 MHz): δ (ppm) = 4.5 - 3.9 (-OCH₂-, 4H), 3.4 - 3.2 (CH, 1H), 2.8 (-CH₂CH₂-, 4H), 1.9 (CH₃, 6H), 1.3 (-OCH₂CH₃, 6H), 1.2 (-CH₃, 18H). ³¹P-NMR (CDCl*₃*, 400 MHz): δ (ppm) = 24.8

### Example 3:

### Synthesis of bis(3-aminopropyl)-terminated poly(dimethyl siloxane) (NH₂-PDMS₁₀₇-NH₂) (n = 107, M_{n,NMR}= 8000 g·mol⁻¹)

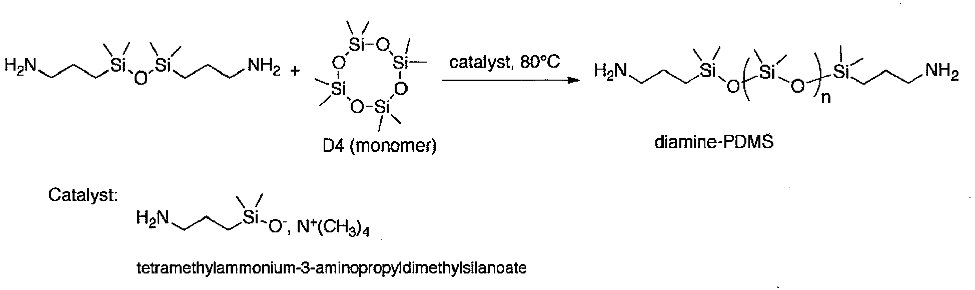

In a 250 mL round bottom flask equipped with a magnetic stirrer, a mixture of 1,3-bis(3-aminopropyl)tetramethyldisiloxane (5.00 g, 83%, 16.67 mmol) and octamethylcyclotetrasiloxane (D4, 12.00 g, 40 mmol) was stirred and heated to 80°C. Tetramethylammonium-3-aminopropyldimethylsilanoate (25.0 mg) as a catalyst was rapidly added to the reaction mixture. The reaction was stirred at 80°C under argon for 30 min. Argon-saturated octamethylcyclotetrasiloxane (136.0 g, 0.459 mol) was then slowly added in 7 h. The reaction mixture was stirred at 80°C for 18 h and then heated to 150°C for 30 min to decompose the catalyst. Volatile components were removed under vacuum at 70°C to obtain a colorless oil with degree polymerization (*DP*ₙ) equal to 107 and *M_{n,NMR}=* 8000 g·mol⁻¹. ¹H-NMR (CDCl*₃*, 400 MHz): δ (ppm) = 2.6 (-NCH₂-, 4H), 1.4 (CH₂, 4H), 0.4 (-CH₂Si, 4H), 0.0 (SiCH₃).

### Example 4:

### Synthesis of α,ω-alkoxyamine-terminated poly(dimethyl siloxane) (MAMASG1-PDMS₁₀₇-MAMASG1) (n =107)

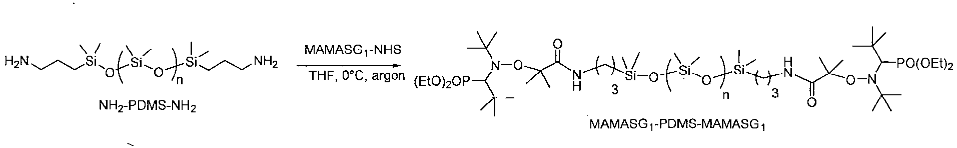

In a 250 mL round bottom flask equipped with a magnetic stirrer, α,ω-diamine-terminated poly(dimethyl siloxane) (NH₂-PDMS₁₀₇-NH₂, *M*ₙ= 8000 g·mol⁻¹, 30.00 g, 3.75 mmol, 1 equiv.) and THF (70.0 mL) were subsequently introduced and stirred under argon at room temperature. The solution was cooled at 0°C using an ice bath. Then, the solution of MAMASG1-NHS (3.67 g, 7.68 mmol, 2.05 equiv.) in THF (20.0 mL) was slowly added into the reaction flask. After finishing the addition, the reaction was continuously agitated at 0°C under argon for 4 h. After, the resulting solution was concentrated under reduced pressure to obtain the viscous oil. The crude oil was dissolved in n-pentane (70.0 mL) and filtered over a short pad of aluminum oxide (Al₂O₃). The filtrate is then concentrated under reduced pressure to give a viscous oil (22.56 g).

¹H-NMR (CDCl₃, 400 MHz): δ (ppm) = 4.3 - 3.9 (-POCH₂-, 4H), 3.4 - 3.2 (CH, 1H), 3.4 - 2.9 (-CH₂NH, 4H), 1.7 - 1.5 (CH-CH₃, 6H), 1.5 (CH₂, 4H), 1.25 (POCH₂CH₃, 6H), 1.1 (CH₃, 18H), 0.0 (Si-CH₃). ³¹P-NMR (CDCl₃, 400 MHz): δ (ppm) = 27.5

### Example 5:

### Synthesis of mono alkoxyamine-terminated poly(dimethyl siloxane) (PDMS₂₅-MA-MASG1) (n = 25)

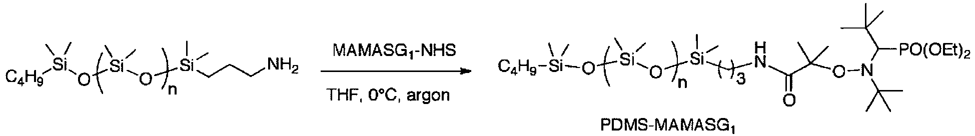

In a 250 mL round bottom flask equipped with a magnetic stirrer, amino-terminated poly(dimethyl siloxane) (Gelest, MCR-A12, *M*ₙ= 2000 g·mol⁻¹, 8.00 g, 4.00 mmol, 1 equiv.) and THF (40.0 mL) were introduced and stirred under argon at room temperature. The solution was cooled at 0°C using an ice bath. Then, the solution of MA-MASG1-NHS (2.00 g, 4.18 mmol, 1.05 equiv.) in THF (25.0 mL) was slowly added into the reaction flask. After finishing the addition, the reaction was continuously agitated at 0°C under argon for 6 h. After, the resulting solution was concentrated under reduced pressure to obtain the viscous oil. The crude oil was dissolved in n-pentane (70.0 mL) and filtered over a short pad of aluminum oxide (Al₂O₃). The filtrate is then concentrated under reduced pressure to give a viscous oil

¹H-NMR (CDCl₃, 400 MHz): δ (ppm) = 4.3 - 3.9 (-POCH₂-, 4H), 3.4 - 3.2 (CH, 1H), 3.4 - 2.9 (-CH₂NH, 2H), 1.7-1.5 (CH-CH₃, 6H), 1.5 (CH₂,4H), 1,25 (POCH₂CH₃, 6H), 1.1 (CH₃, 18H), 0.7 (-CH₂CH₂CH₃, 7H), 0.2 (CH₂Si, 2H), 0.0 (Si-CH₃). ³¹P-NMR (CDCl₃, 400 MHz): δ (ppm) = 27.5

### Example 6:

### Synthesis of triblock copolymer P(MMA-co-S)-b-PDMS₁₀₇-b-P(MMA-co-S) (BCP1, n = 107, p = 3, q = 52)

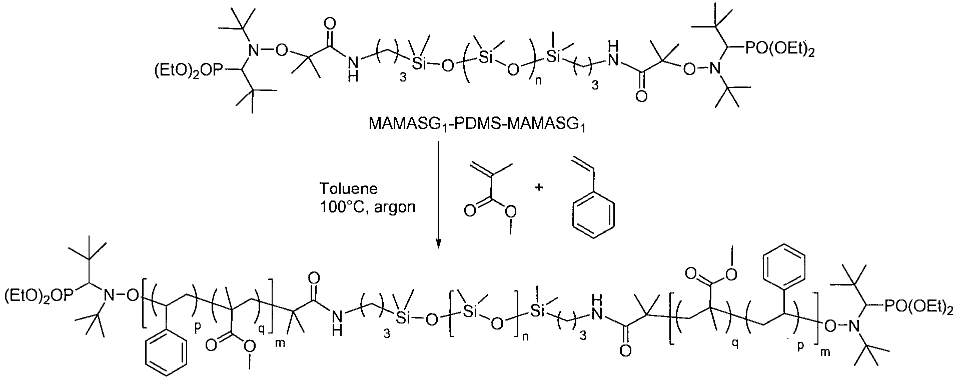

MAMASG1-PDMS₁₀₇-MAMASG1 (Example 4) (*M*_{n,NMR}= 8730 g·mol⁻¹, 10.00 g, 1.15 mmol), methyl methacrylate (MMA, 16.0 g, 160 mmol), styrene (0.88 g, 8.46 mmol), and toluene (14.0 mL) were charged to a round bottom flask equipped with a magnetic stir bar. The mixture was deoxygenated by bubbling argon for 40 min at 0°C with an ice bath. The solution was then immersed in an oil bath thermostated at 100 °C to allow the polymerization to occur. After 180 min, the polymerization was quenched by rapid cooling and exposure of the polymerization solution to air. The resulting product was then dissolved in dichloromethane and precipitated in methanol, filtered, and dried under vacuum to obtain the final polymer as a white solid with number average molecular weight determined by size exclusion chromatography (SEC) *M*_{n,SEC} = 26400 g·mol⁻¹, and dispersity *Ð*= 1.45.

Dispersity or molecular weights distribution is the ratio between weight-average molecular weight (M_{w}) and number-average molecular weight (Mₙ) (*Ð*=M_{w}/Mₙ). Size exclusion chromatography (SEC) analyses were performed on an EcoSEC apparatus from PSS, equipped with a dual flow cell refractive index detector. Eluent was THF at a flow rate of 0.3 mL·min⁻¹ for the sample pump and 0.15 mL·min⁻¹ for the reference pump. The stationary phase was a combination of one PL Resipore (50x4.6) mm guard column and two PL Resipore (250x4.6) mm columns thermostated at 40°C. Samples were prepared by dissolving 10 mg of sample in 2 mL of THF containing 0.25 vol.% of toluene, as a flowmarker. The soluble fraction of sample was filtered through 0.45 µm PTFE filters. Injection volume was 20 µL. Poly(methyl methacrylate) (PMMA) equivalent number-average and weight-average molar masses (*M*ₙ and *M*_{w} respectively) and dispersities *Ð* were calculated by means of a PMMA calibration curve using PMMA standards.

¹H-NMR (CDCl₃, 400 MHz): δ (ppm) = 7.2 - 6.8 (ArH), 3.6 (OCH₃), 2.9 (-CH₂NH), 2.2 - 1.5 (CH₂ of main chain), 1.5 - 0.5 (remaining protons), 1.0 - 0.7 (CCH₃), 0.0 (Si-CH₃).

### Example 7:

### Synthesis of triblock copolymer P(MMA-co-S)-b-PDMS₁₀₇-b-P(MMA-co-S) (BCP2, n =107, p = 2, q = 35)

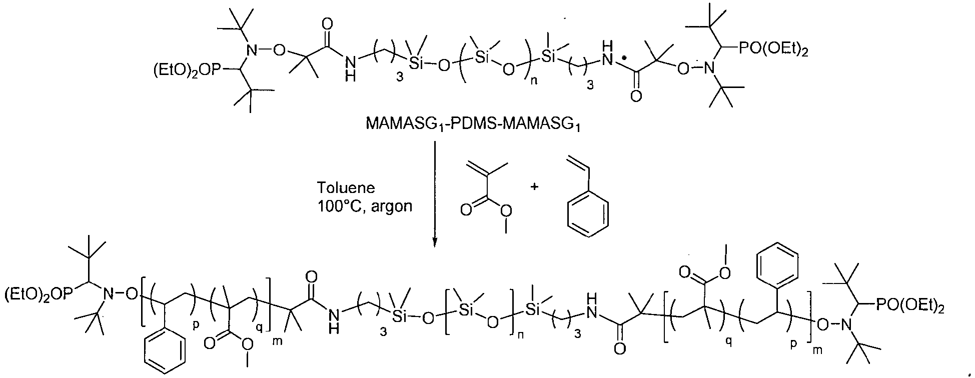

MAMASG1-PDMS107-MAMASG1 (Example 4) macroinitiator (*M*_{n,NMR}= 8730 g·mol⁻¹, 18.00 g, 2.06 mmol), methyl methacrylate (MMA, 21.0 g, 210 mmol), styrene (1.08 g, 10.38 mmol), and toluene (22.0 mL) were charged to a round bottom flask equipped with a magnetic stir bar. The mixture was deoxygenated by bubbling argon for 40 min at 0°C with an ice bath. The solution was then immersed in an oil bath thermostated at 100 °C to allow the polymerization to occur. After 240 min, the polymerization was quenched by rapid cooling and exposure of the polymerization solution to air. The resulting product was then dissolved in dichloromethane and precipitated in methanol, filtered, and dried under vacuum to obtain the final polymer as a white solid with *M*_{n,SEC}= 22900 g·mol⁻¹, and *Ð*= 1.29.

¹H-NMR (CDCl₃, 400 MHz): δ (ppm) = 7.2 - 6.8 (ArH), 3.5 (OCH₃), 2.9 (-CH₂NH), 2.1 1.5 (CH₂ of main chain), 1.5 - 0.5 (remaining protons), 1.0 - 0.7 (CCH₃), 0 (Si-CH₃).

### Example 8:

### Synthesis of triblock copolymer P(BMA-co-S)-b-PDMS₁₀₇-b-P(BMA-co-S) (BCP3, n = 106, p = 5, q = 54)

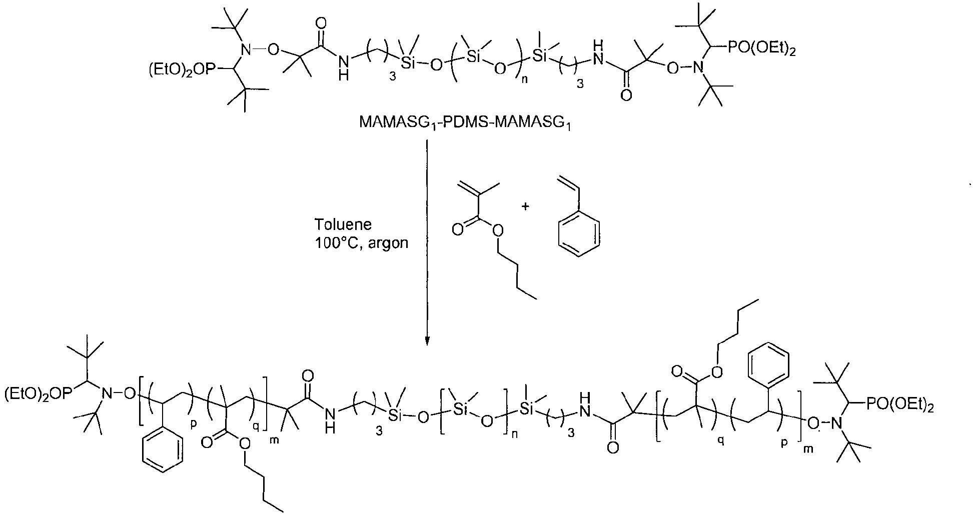

MAMASG1-PDMS107-MAMASG1 (Example 4) macroinitiator (*M*_{n,NMR}= 8730 g·mol⁻¹, 18.00 g, 2.06 mmol), *n*-butyl methacrylate (BMA, 28.00 g, 197.2 mmol), styrene (1.08 g, 10.38 mmol), and toluene (23.0 mL) were charged to a round bottom flask equipped with a magnetic stir bar. The mixture was deoxygenated by bubbling argon for 40 min at 0°C with an ice bath. The solution was then immersed in an oil bath thermostated at 100 °C to allow the polymerization to occur. After 180 min, the polymerization was quenched by rapid cooling and exposure of the polymerization solution to air. The resulting product was then dissolved in dichloromethane and precipitated in cooled methanol, filtered, and dried under vacuum to obtain the final polymer as a white solid with *M*_{n,SEC}= 24100 g·mol⁻¹, and *Ð*= 1.29.

¹H-NMR (CDCl₃, 400 MHz): δ (ppm) = 7.2 - 6.8 (ArH), 3.9 (OCH₂), 3.1 (-CH₂NH), 2.2 - 1.6 (CH₂ of main chain), 1.6 - 1.3 (OCH₂-CH₂CH₂-CH₃), 1.3 - 0.5 (remaining protons), 1.0 - 0.7 (CCH₃ and CH₃ of side group), 0.0 (Si-CH₃).

### Example 9:

### Synthesis of triblock copolymer P(EMA-co-S)-b-PDMS₁₀₇-b-P(EMA-co-S) (BCP4, n = 107, p = 5, q = 54)

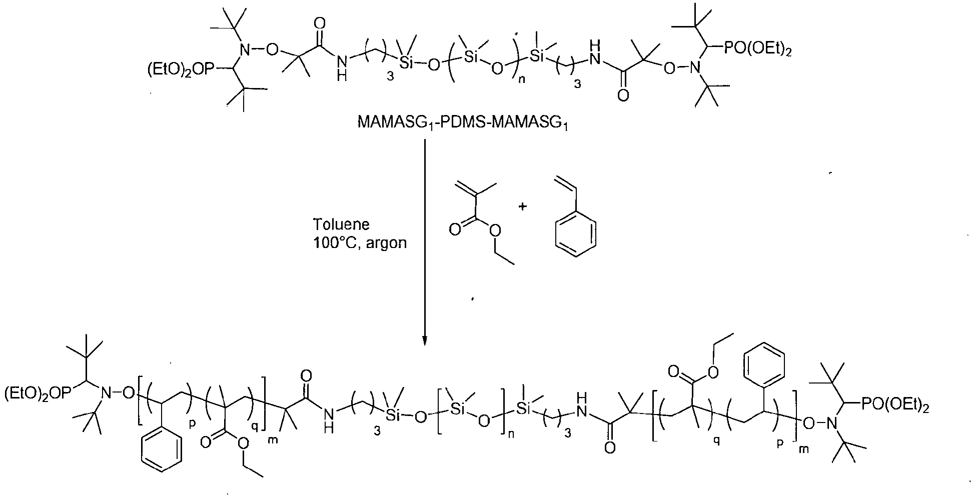

MAMASG1-PDMS107-MAMASG1 (Example 4) (*M*_{n,NMR}= 8730 g·mol⁻¹, 10.00 g, 1.15 mmol), ethyl methacrylate (EMA, 26.00 g, 228.1 mmol), styrene (1.40 g, 13.46 mmol), and toluene (19.0 mL) were charged to a round bottom flask equipped with a magnetic stir bar. The mixture was deoxygenated by bubbling argon for 40 min at 0°C with an ice bath. The solution was then immersed in an oil bath thermostated at 100°C to allow the polymerization to occur. After 300 min, the polymerization was quenched by rapid cooling and exposure of the polymerization solution to air. The resulting product was then dissolved in dichloromethane and precipitated in cooled methanol, filtered, and dried under vacuum to obtain the final polymer as a white solid with *M*_{n,SEC}= 47900 g·mol⁻¹, and *Ð*=1.33.

¹H-NMR (CDCl₃, 400 MHz): δ (ppm) = 7.2 - 6.8 (ArH), 4.0 (OCH₂), 3.1 (-CH₂NH), 2.2 - 1.6 (CH₂ of main chain), 1.2 (CH₃ of side group), 1.3 - 0.5 (remaining protons), 0.9 - 0.7 (CCH₃), 0.0 (Si-CH₃)

### Example 10:

### Synthesis of diblock copolymer PDMS-b-P(MMA-co-S) (BCP5; n = 25 and p = 3, q = 29)

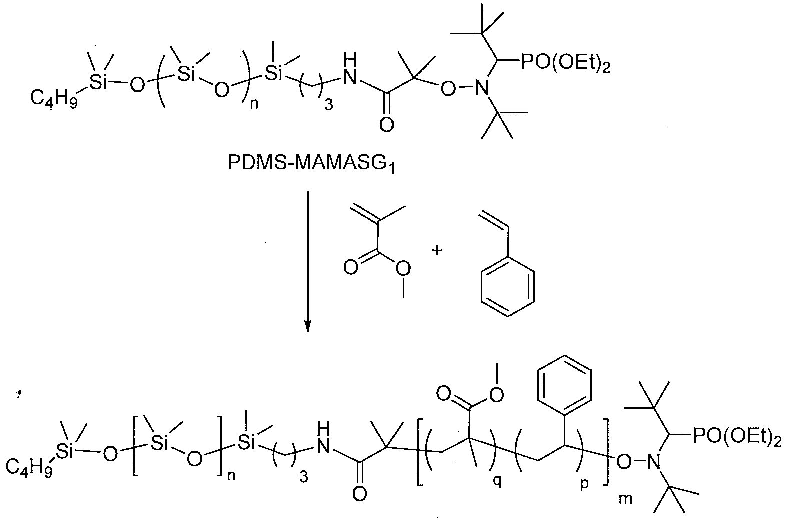

Alkoxyamine-terminated poly(dimethyl siloxane) (PDMS₂ₖ-MAMASG1, *M_{n,NMR}=* 2380 g·mol⁻¹, 10.00 g, 4.20 mmol), and methyl methacrylate (MMA, 16.0 g, 160 mmol), styrene (0.80 g, 7.69 mmol) and toluene (10.0 mL) were charged to a round bottom flask equipped with a magnetic stir bar. The mixture was deoxygenated by bubbling argon for 40 min at 0°C with an ice bath. The solution was then immersed in an oil bath thermostated at 100 °C to allow the polymerization to occur. After 140 min, the polymerization was quenched by rapid cooling and exposure of the polymerization solution to air. The resulting product was then dissolved in dichloromethane and precipitated in cooled methanol, and dried under vacuum to obtain the final polymer as a white solid with *M*_{n,SEC}= 10100 g·mol⁻¹, *Ð*= 1.29.

¹H-NMR (CDCl*₃*, 400 MHz): δ (ppm) = 7.2 - 6.8 (ArH), 3.5 (OCH₃), 2.9 (-CH₂NH), 2.1 - 1.5 (CH₂ of main chain), 1.5 - 0.5 (remaining protons), 1.0 - 0.7 (CCH₃), 0.0 (Si-CH₃).

### Example 11:

### Synthesis of triblock copolymer P(PDGMEM-co-S)-b-PDMS₁₀₇-b-P(PDGMEM-co-S) (BCP6, n = 107, p = 1, q = 11)

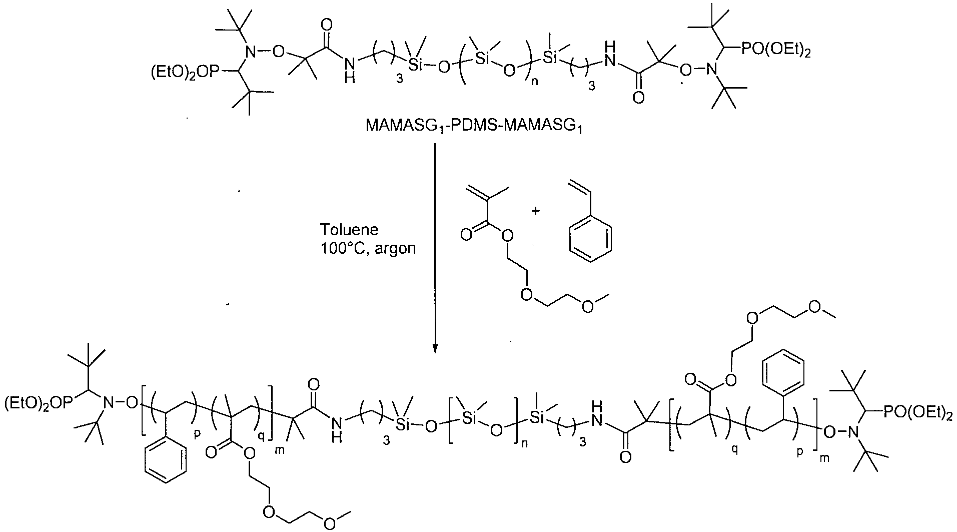

MAMASG1-PDMS107-MAMASG1 (Example 4) (*M*_{n,NMR}= 8730 g·mol⁻¹, 5.00 g, 0.575 mmol), di(ethylene glycol) methyl ether methacrylate (DGMEM, 9.20 g, 48.85 mmol), styrene (0.51 g, 4.88 mmol), and toluene (7.5 mL) were charged to a round bottom flask equipped with a magnetic stir bar. The mixture was deoxygenated by bubbling argon for 40 min at 0°C with an ice bath. The solution was then immersed in an oil bath thermostated at 100°C to allow the polymerization to occur. After 120 min, the polymerization was quenched by rapid cooling and exposure of the polymerization solution to air. The resulting product was then dissolved in tetrahydrofuran and precipitated in water, filtered, and dried under vacuum to obtain the final polymer as a white solid with *M*_{n,SEC}= 26790 g·mol⁻¹, and *Ð*=1.29. ¹H-NMR (CDCl₃, 400 MHz): δ (ppm) = 7.2 - 6.9 (ArH), 4.12 (OCH₂), 3.72-3.55 (OCH₂), 3.35 (OCH₃), 2.1 - 1.5 (CH₂ of main chain), 1.5 - 0.5 (remaining protons), 0.9 - 0.7 (CCH₃), 0.12 (Si-CH₃)

### Example 12:

### Synthesis of triblock copolymer P(PGMEM-co-S)-b-PDMS₁₀₇-b-P(PGMEM-co-S) (BCP7, n =107, ρ = 3, g = 34)

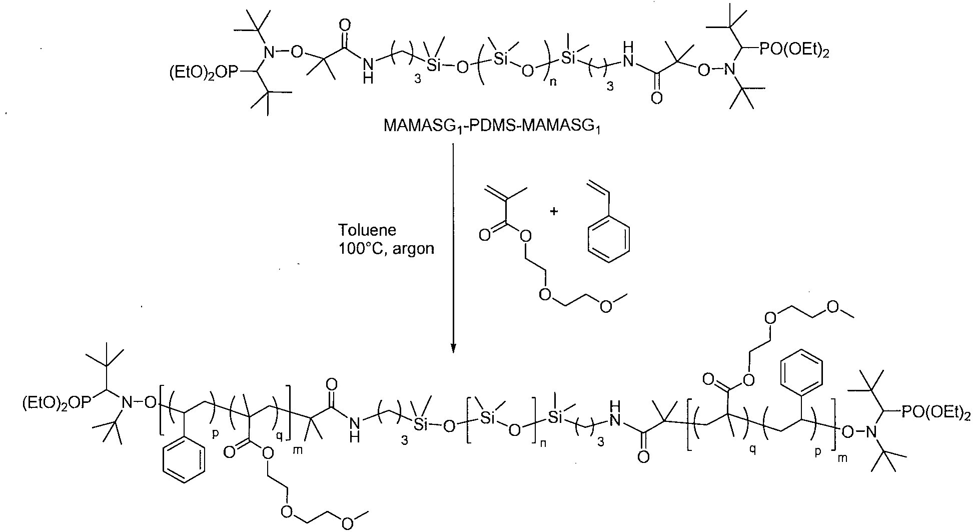

MAMASG1-PDMS107-MAMASG1 (Example 4) (*M*_{n,NMR}= 8730 g·mol⁻¹, 5.00 g, 0.575 mmol), di(ethylene glycol) methyl ether methacrylate (27.60 g, 146.6 mmol), styrene (1.525 g, 14.66 mmol), and toluene (17.0 mL) were charged to a round bottom flask equipped with a magnetic stir bar. The mixture was deoxygenated by bubbling argon for 40 min at 0°C with an ice bath. The solution was then immersed in an oil bath thermostated at 100°C to allow the polymerization to occur. After 120 min, the polymerization was quenched by rapid cooling and exposure of the polymerization solution to air. The resulting product was then dissolved in tetrahydrofuran and precipitated in water, filtered, and dried under vacuum to obtain the final polymer as a white solid with *M*_{n,SEC}= 36400 g·mol⁻¹, and *Ð*=1.36. ¹H-NMR (CDCl₃, 400 MHz): δ (ppm) = 7.2 - 6.9 (ArH), 4.12 (OCH₂), 3.72-3.55 (OCH₂), 3.35 (OCH₃), 2.1 - 1.5 (CH₂ of main chain), 1.5 - 0.5 (remaining protons), 0.9 - 0.7 (CCH₃), 0.12 (Si-CH₃).

### Example 13:

### Synthesis of triblock copolymer PCL-PDMS-PCL (BCP8, n = 107) (4000-8000-4000)

Under an inert atmosphere, a mixture of α,ω-diamine-terminated poly(dimethyl siloxane) (NH₂-PDMS₁₀₇-NH₂, *M*ₙ= 8000 g·mol⁻¹, 30.00 g) and *ε*-caprolactone (10.20 g) was heated to 80 °C. After 30 min, tin(II) 2-ethylhexanoate (20 mg) was added and the temperature was gradually raised to 130 °C within 30 min. After 22 h, volatiles were removed under reduced pressure (0.3 mbar, 130 °C - 160 °C), affording 19.57 g (97%) of the block copolymer as a rubbery yellowish solid.

¹H NMR (CDCl₃, 400 MHz): δ = 0.15 (s, 620H; Si-CH₃); 0.58-0.64 (m, 4H; Si-CH₂); 1.41-1.53 (m, 134H; CH₂); 1.64-1.81 (m, 268H; CH₂); 2.25 (t, 4H; *J =* 7.5 Hz; N-CH₂); 2.39 (t, 130H; *J* = 7.5 Hz; C(O)-CH₂); 3.30 (q, 4H; *J* = 6.7 Hz; N-CH₂); 3.72 (t, 4H; *J* = 6.7 Hz; HO-CH₂); 4.14 (t, 130H; *J* = 6.7 Hz; O-CH₂).

### Example 14:

### Formulation and evaluation of (meth)acrylate-based materials containing BCP1 or BCP2

Resins R1-9 were prepared by homogeneously mixing the compound listed in Table 1. TPO was used as a photoinitiator. DMA1 from Example 1 was used as urethane dimethacrylate and PEMA (2-phenoxyethyl methacrylate) as monofunctional monomer. Resin R1 did not contain any block copolymer (BCP) and was used as a first reference. Resins R8 and R9 contained the known PCL(polycaprolactone)-PDMS-PCL (1600 g/mol - 3200 g/mol - 1600 g/mol) BCP (PO-277) and were used as a second reference.

**Table 1: Composition of resins R1-10**

| **Component** | **R1*** | **R2** | **R3** | **R4** | **R5** | **R6** | **R7** | **R8*** | **R9*** | **R10** |
|---|---|---|---|---|---|---|---|---|---|---|
| DMA1 | 44.55 | 43.20 | 42.30 | 43.20 | 42.30 | 42.30 | 42.30 | 43.20 | 42.30 | 42.30 |
| PEMA | 54.45 | 52.80 | 51.70 | 52.80 | 51.70 | 51.70 | 51.70 | 52.80 | 51.70 | 51.70 |
| TPO | 1.0 | 1.0 | 1.0 | 1.0 | 1.0 | 1.0 | 1.0 | 1.0 | 1.0 | 1.0 |
| BCP1 | - | 3.0 | 5.0 | - | - | - | - | - | - | - |
| BCP2 | - | - | - | 3.0 | 5.0 | - | - | - | - | - |
| BCP6 | - | - | - | - | - | 5.0 | - | - | - | - |
| BCP7 | - | - | - | - | - | - | 5.0 | - | - | - |
| PO-277¹⁾ | - | - | - | - | - | - | - | 3.0 | 5.0 | - |
| BCP8 | - | - | - | - | - | - | - | - | - | 5.0 |

| | | | | | | | | | | |
|---|---|---|---|---|---|---|---|---|---|---|
| * comparative example ¹⁾ PCL(1600)-b-PDMS(3200)-PCL(1600) BCP (Ex. 3 of EP 4 085 893 A1) | | | | | | | | | | |

### Example 15:

### Testing of flexural strength and fracture toughness

The flexural strength of the materials described in Example 13 was measured both dry and in a water bath after storage in water.

According to the test procedures of ISO4049:2019, specimens with the dimensions of 25 mm x 2 mm x 2 mm were prepared by light curing of the resins using a stainless-steel mould, covered by a 50 µm thick polyester film to prevent oxygen inhibition. Light curing of the specimens was achieved by placing them in a LED light curing device (PrograPrint^{®} Cure; Ivoclar, Schaan, Liechtenstein) and curing them using the program "Splint" (90s, 405 nm wavelength, light intensity 120 mW/cm²) from both the top and bottom sides. After curing, excess material was removed by careful sanding of all faces (320 grit paper) and specimens were stored for 24h at 37°C in water. After water storage, the specimens were measured dry at 23°C in a universal testing machine in a 3-point-bending test (20 mm span) at a crosshead speed of 0.8 mm/min.

In addition, the flexural strength was measured in a water bath at 37°C to simulate mouth conditions. Test specimens were prepared according to ISO4049:2019 as described above. After water storage, the specimens were fixed to the test jig of the 3-point-bending-test device and then submerged in a water bath at 37°C for measurement.

Fracture toughness was measured in analogy to ISO20795-1:2013. Prior to the measurement, the specimens were stored in water at 37°C for a shortened period of 24h. All other procedures and parameters were kept identical to ISO20795.1 :2013. Light curing of the specimens was achieved by placing them in a PrograPrint^{®} Cure device (Ivoclar, Schaan, Liechtenstein) and curing them using the program "Splint" (90s, 405 nm wavelength, light intensity 120 mW/cm²) from both the top and bottom sides.

The results of all measurements are shown in Table 2.

The cured resin R1, that does not contain any block copolymer, exhibits good flexural strength when measured in a water bath at 37°C (FS is greater than 60 MPa). However, this material is brittle and has a low fracture toughness (Kₘₐₓ is significantly lower than 1.2 MPa·m^{1/2}).

The reference materials R8 and R9, containing different amounts of the PCL-PDMS-PCL block copolymer PO-277, exhibit significantly higher Kₘₐₓ values than R1 (Kₘₐₓ is greater than1.2 Mpa·m^{1/2}). These results confirm that PO-277 is an efficient toughening agent. However, the flexural strength measured under oral conditions is relatively low (FS is lower than 60 MPa). Similar results were obtained with BCP8 (same PDMS block length as triblock copolymers BCP1, BCP2, BCP6 and BCP7).

The materials containing the block copolymers BCP1, BCP2, BCP6 and BCP7 according to the present invention had excellent physical properties. The addition of these BCPs to the methacrylate mixture led to a strong improvement of the fracture toughness without significantly impairing the mechanical properties. These materials exhibit good values for flexural strength under oral conditions (FS is greater than 60 MPa, measured in a water bath at 37°C) and also for fracture toughness (Kₘₐₓ is greater than 1.2 MPa·m^{1/2}), which is a considerable advantage for the intended use as a dental material.

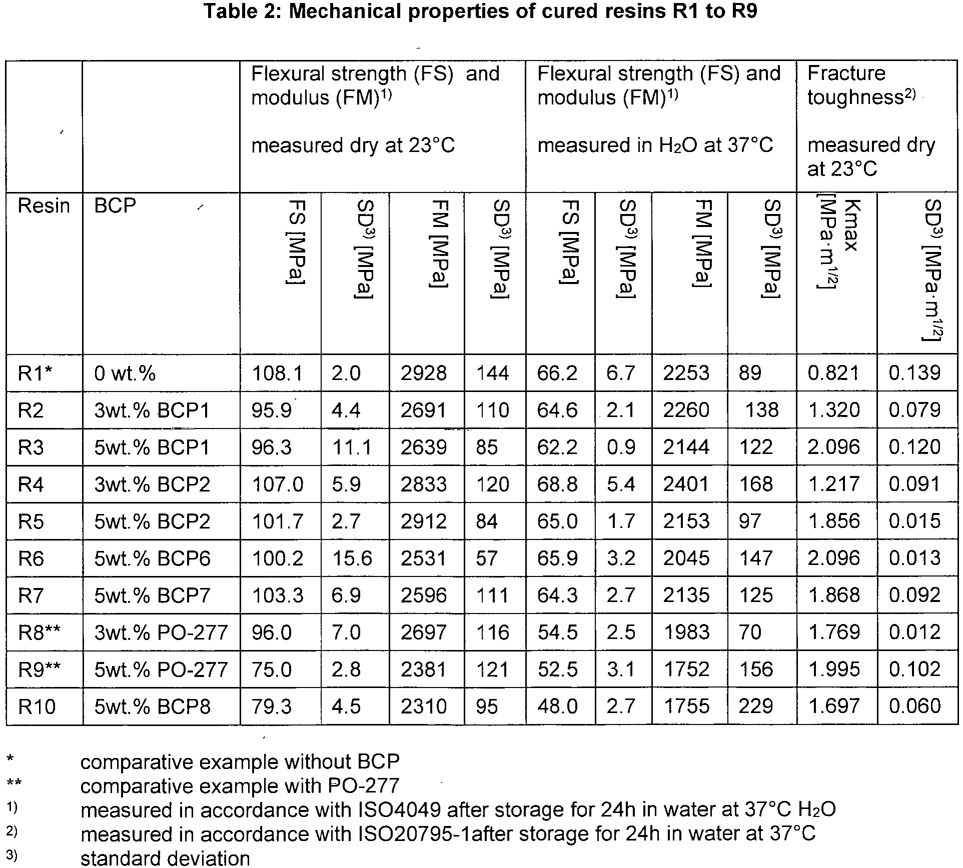

## Claims

1. Block copolymer according to Formula 1, 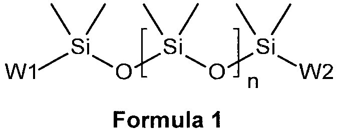 **characterized in that**
n is a value of from 5 to 250, preferably from 10 to 200, more preferably from 15 to 150;
W1 is a group according to Formula 2, which is attached to the Si atom of Formula 1 via X¹, 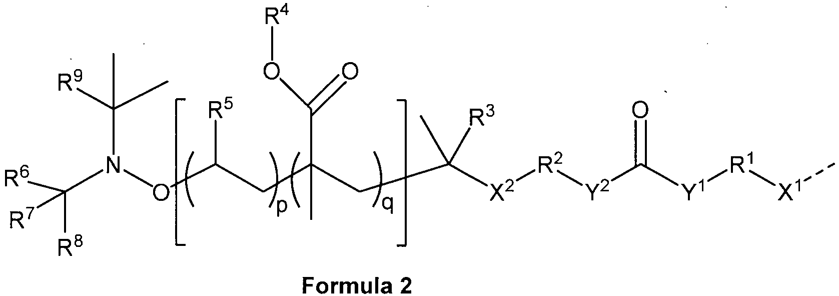
wherein
X¹ is CH₂ or an O atom;
X² is a -C(O)O- group or is absent;
Y¹ is absent, an O atom, a NR²¹ group in which R²¹ is a hydrogen atom or a branched or a linear C₁-C₁₂ alkyl group, preferably a hydrogen atom or a C₁-C₈ alkyl group, and more preferably a hydrogen atom or a C₁-C₆ alkyl group, whereby Y¹ is absent if Y² is an O atom;
Y² is an O atom or is absent, whereby Y² is absent if Y¹ is an O atom or a NR²¹ group;
R¹ is a branched or linear C₁-C₁₆ alkanediyl group, preferably a branched or linear C₁-C₁₂ alkanediyl group, and more preferably a branched or linear C₁-C₆ alkanediyl group, that is interrupted by one or more O atoms and/or -C(O)O- groups and is preferably uninterrupted;
R² is absent or a branched or a linear C₁-C₁₀ alkanediyl group, preferably a branched or linear C₁-C₈ alkanediyl group, and more preferably a branched or linear C₁-C₆ alkanediyl group, that is uninterrupted or interrupted by one or more, preferably one, O atoms and/or -C(O)O- groups whereby R² cannot be absent if X² is a -C(O)O- group;
R³ is hydrogen, a methyl, nitrile or -(C=O)OR¹⁰ group, preferably a methyl or nitrile group, and R¹⁰ is a branched or a linear C₁-C₁₂ alkyl group, preferably a branched or a linear C₁-C₈ alkyl group, and more preferably a branched or a linear C₁-C₆ alkyl group;
R⁴ is a branched or preferably linear C₁-C₁₂ alkyl group, preferably a branched or preferably linear C₁-C₁₀ alkyl group, and more preferably a branched or preferably linear C₁-C₈ alkyl group, that is uninterrupted or interrupted by one or more, preferably 1 to 3, and more preferably 1 to 2, S atoms, O atoms, -C(O)O- groups and/or -C(O)NH- groups, and which is unsubstituted or substituted by one or more, preferably one, functional groups, preferably OH, COOH and/or halogen;
or
a C₄-C₁₆ cycloaliphatic group that is unsubstituted or substituted by one or more, preferably 1 to 5, and more preferably 1 to 3, substituents selected from branched or preferably linear C₁-C₂₀ alkyl groups, preferably branched or preferably linear C₁-C₁₂ alkyl groups, more preferably branched or preferably linear C₁-C₆ alkyl groups, and that is uninterrupted or interrupted by one or more, preferably 1 to 4, and more preferably 1 to 2, S atoms, O atoms, -C(O)O- groups and/or C(O)NH- groups;
or
a C₄-C₁₀ aryl or heteroaryl group which is unsubstituted or substituted by one or more, preferably 1 to 4, and more preferably 1 to 2, substituents selected from branched or preferably linear C₁-C₂₀ alkyl groups, -O-C₁-C₁₂ alkoxy groups, ester groups -C(O)O-R¹¹, and/or acyl groups -C(O)-R¹², wherein R¹¹ and R¹² are each independently of one other a branched or preferably linear C₁-C₂₀ alkyl group, a C₆-C₁₀ aryl group or a C₇-C₂₀- alkylaryl group, and can be interrupted by one or more S atoms or O atoms and are preferably uninterrupted;
or
a C₅-C₂₀-alkylaryl or C₅-C₂₀-alkylheteroaryl group that is uninterrupted or interrupted by one or more, preferably 1 to 2, and more preferably 1 S atoms, O atoms, -C(O)O- groups and/or -C(O)NH- groups, and that is unsubstituted or substituted by one or more, preferably 1 to 4, and more preferably 1 to 2, substituents selected from C₁-C₂₀ alkyl groups, branched or preferably linear -O-C₁-C₁₂ alkoxy groups, branched or preferably linear ester groups -C(O)O-R¹³, and/or branched or preferably linear acyl groups -C(O)-R¹⁴, where R¹³ is a branched or preferably linear C₁-C₂₀ alkyl group, a C₆-C₁₀ aryl group or a C₇-C₂₀-alkylaryl group, and R¹⁴ is a branched or preferably linear C₁-C₂₀ alkyl group, a C₆-C₁₀ aryl group or a C₇-C₂₀-alkylaryl group;
R⁵ is a phenyl group or a -(C=O)OR¹⁵ group in which
R¹⁵ is a branched or preferably linear C₁-C₁₂ alkyl group, preferably a branched or preferably linear C₁-C₁₀ alkyl group, and more preferably a branched or preferably linear C₁-C₈ alkyl group, that is uninterrupted or interrupted by one or more, preferably 1 to 4, and more preferably 1 to 2, S atoms, O atoms, -C(O)O- groups, and/or -C(O)NH- groups, and that is unsubstituted or is substituted by one or more functional groups, preferably OH, COOH and/or halogen;
or
a C₄-C₁₆ cycloaliphatic group that is unsubstituted or substituted by one or more, preferably 1 to 5, and more preferably 1 to 3, substituents selected from branched or preferably linear C₁-C₂₀ alkyl groups, preferably from branched or preferably linear C₁-C₁₂ alkyl groups, more preferably from branched or preferably linear C₁-C₆ alkyl groups, and that is uninterrupted or interrupted by one or more, preferably 1 to 4, and more preferably 1 to 2, S atoms, O atoms, -C(O)O- groups and/or C(O)NH- groups;
or
a C₄-C₁₀ aryl or heteroaryl group that is unsubstituted or substituted by one or more, preferably 1 to 4, and more preferably 1 to 2, substituents which are selected from branched or preferably linear C₁-C₂₀ alkyl groups, branched or preferably linear -O-C₁-C₁₂ alkoxy groups, branched or preferably linear ester groups -C(O)OR¹⁶, and/or branched or preferably linear acyl groups -C(O)-R¹⁷, and that is uninterrupted or interrupted by one or more S atoms or O atoms, where R¹⁶ is a branched or preferably linear C₁-C₂₀ alkyl group, a C₆-C₁₀ aryl group or a C₇-C₂₀-alkylaryl group, and R¹⁷ is a branched or preferably linear C₁-C₂₀ alkyl group, a C₆-C₁₀ aryl group or a C₇-C₂₀-alkylaryl group,
or
a C₅-C₂₀-alkylaryl or C₅-C₂₀-alkylheteroaryl group that is uninterrupted or interrupted by one or more, preferably 1 to 2, and more preferably 1, S atoms, O atoms, -C(O)O- groups and/or -C(O)NH- groups, and that is unsubstituted or substituted by one or more, preferably 1 to 4, and more preferably 1 to 2, substituents selected from branched or preferably linear C₁-C₂₀ alkyl groups, branched or preferably linear O-C₁-C₁₂ alkoxy groups, branched or preferably linear ester groups -C(O)O-R¹⁸, and/or branched or preferably linear acyl groups -C(O)-R¹⁹, where R¹⁸ is a branched or preferably linear C₁-C₂₀ alkyl group, a C₆-C₁₀ aryl group or a C₇-C₂₀-alkylaryl group, and R¹⁹ is a branched or preferably linear C₁-C₂₀ alkyl group, a C₆-C₁₀ aryl group or a C₇-C₂₀-alkylaryl group;
R⁶ is a hydrogen atom, a methyl group, or forms together with R⁹ a -CH₂-CHR²²-CH₂- group, wherein R²² is H or an OH group;
R⁷ is a methyl group, a -CH(CH₃)₂ group or a -C(CH₃)₃ group;
R⁸ is a methyl group, preferably a phenyl group or a P(O)(OR²⁰)₂ group in which R²⁰ is a branched or preferably linear C₁-C₁₂ alkyl group, preferably a branched or preferably linear C₁-C₆ alkyl group, and more preferably a branched or preferably linear C₁-C₄ alkyl group;
R⁹ is a methyl group or forms together with R⁶ a -CH₂-CHR²²-CH₂- group, wherein R²² is H or an OH group;
p is a number of from 1 to 400, preferably from 1 to 200, more preferably from 1 to 140;
q is a number of from 0 to 400, preferably from 0 to 200, more preferably from 0 to 140;
p + q is > 5;
W2 is a group according to Formula 2, which is attached to the Si atom of Formula 1 via X¹, as indicated by the dashed line, or is a branched or preferably linear C₁-C₁₂ alkyl group, preferably a group according to Formula 2 or a branched or preferably linear C₁-C₆ alkyl group, and more preferably a group according to Formula II or a branched or preferably linear C₁-C₄ alkyl group.

2. The block copolymer according to claim 1, wherein
p/(p+q) is a value of from 0.01 to 0.5, preferably from 0.02 to 0.2, more preferably from 0.04 to 0.15, if R⁵ is a phenyl group; and is a value of from 0.01 to 1.0, preferably from 0.02 to 1.0, more preferably from 0.04 to 1.0, if R⁵ is a -(C=O)OR¹⁵ group.

3. The block copolymer according to claim 1 or 2, wherein
n is a value of from 15 to 150;
W1 is a group according to Formula 2, wherein
X¹ is CH₂;
X² is absent;
Y¹ is a NR²¹ group in which R²¹ is a hydrogen atom;
Y² is absent;
R¹ is a linear C₁-C₄ alkanediyl group;
R² is absent;
R³ is a methyl group;
R⁴ is a linear C₁-C₈ alkyl group, that is uninterrupted or interrupted by 1 to 2, O atoms;
R⁵ is a phenyl group or a -(C=O)OR¹⁵ group in which
R¹⁵ is a branched or preferably linear C₁-C₈ alkyl group
or
a C₄-C₁₆ cycloaliphatic group that is unsubstituted or substituted by one or more, preferably 1 to 5, and more preferably 1 to 3, branched or preferably linear C₁-C₆ alkyl groups, or
a C₄-C₁₀ aryl or heteroaryl group
or
a C₅-C₂₀-alkylaryl or C₅-C₂₀-alkylheteroaryl group
R⁶ is a hydrogen atom;
R⁷ is a -CH(CH₃)₂ group or a -C(CH₃)₃ group;
R⁸ is a P(O)(OR²⁰)₂ group in which R²⁰ is a phenyl group or preferably a linear C₁-C₄ alkyl group, more preferably an ethyl group;
R⁹ is a methyl group;
if R⁵ is a phenyl group, p and q have the following meanings:
p is a value of from 1 to 10;
q is a value of from 10 to 140;
p + q is > 5;
p/(p+q) is a value of from 0.04 to 0.15;
if R⁵ is a -(C=O)OR¹⁵ group, p and q have the following meanings:
p is a value of from 6 to 140;
q is a value of from 0 to 140;
p + q is >5;
p/(p+q) is a value of from 0.04 to 1.0;
W2 is a branched or preferably linear C₁-C₄ alkyl group, or preferably is equal to W1.

4. The block copolymer according to claim 1, wherein
n is a number of from 20 to 125;
W1 is a group according to Formula 2, wherein
X¹ is CH₂;
X² is absent;
Y¹ is a NR²¹ group in which R²¹ is a hydrogen atom;
Y² is absent;
R¹ is a linear C₁-C₄ alkanediyl group;
R² is absent;
R³ is a methyl group;
R⁴ is a linear C₁-C₈ alkyl group, that is uninterrupted or interrupted by 1 to 2, O atoms
R⁵ is a phenyl group;
R⁶ is a hydrogen atom;
R⁷ is a -C(CH₃)₃ group;
R⁸ is a P(O)(OR²⁰)₂ group in which R²⁰ is linear C₁-C₄ alkyl group, preferably an ethyl group;
R⁹ is a methyl group;
p is a value of from 1 to 10;
q is a value of from 10 to 140 ;
W2 is equal to W1.

5. A process for synthesizing the block copolymer according to any one of claims 1 to 4 by the following synthesis route, comprising:
(I) synthesizing a poly(dimethylsiloxane) (PDMS) oligomer according to formula 7 or 8, 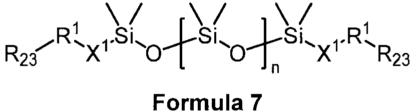 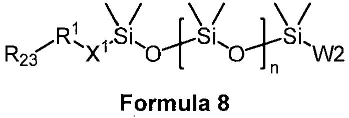 wherein R²³ is a OH, a COOH or a NHR²¹ group, and R²¹ is a hydrogen atom or a branched or a linear C₁-C₁₂ alkyl group, preferably a hydrogen atom or a C₁-C₈ alkyl group, and more preferably a hydrogen atom or a C₁-C₆ alkyl group;
(II) reacting the PDMS oligomer of Formula 7 or 8 with an alkoxyamine bearing a R²⁵ group, wherein R²⁵ is a COOH or a COOR²⁶ group, and wherein
R²⁶ is a 4-nitrophenyl, a 2,3,5,6-tetrafluorphenyl or preferably a N-hydroxysuccinimide group, if R²³ is a OH or a NHR²¹ group,
or
reacting the PDMS oligomer of Formula 7 or 8 with an alkoxyamine bearing a OH group (esterification) if R²¹ is a COOH group;
(III) polymerizing a monofunctional acrylate monomer, or copolymerizing one monofunctional methacrylate and one comonomer, by nitroxide-mediated radical polymerization (NMP) using the PDMS alkoxyamine of Formula 8 as initiators to obtain a block copolymer according to Formula 1.

6. A radically polymerizable material comprising at least one block copolymer according to any one of claims 1 to 4, at least one radically polymerizable monomer and at least one initiator for the radical polymerization.

7. The material according to claim 6, comprising
(a) 30 to 70 wt.%, preferably 30 to 65 wt.% and more preferably 40 to 60 wt.% of at least one aromatic, bicyclic and/or tricyclic mono(meth)acrylate,
(b) 20 to 65 wt.%, preferably 25 to 60 wt.% and more preferably 30 to 55 wt.% of at least one urethane di(meth)acrylate,
(c) 0 to 30 wt.%, preferably 0 to 20 wt.% and more preferably 0 wt.% of one ore more dimethacrylate monomers that do not bear a urethane group,
(d) 1 to 15 wt.%, preferably 2 to 13 wt.% und more preferably 3 to 10 wt.% of the at least one block copolymer according to one of claims 1 to 4,
(e) 0.1 to 5.0 wt.%, preferably 0.2 to 4.0 wt.% and more preferably 0.3 to 3.0 wt.% of at least one photoinitiator,
whereby all percentages refer to the total weight of the dental material.

8. The material according to claim 7, which comprises as component (a) at least one aromatic, bicyclic or tricyclic monomethacrylate of Formula (9): 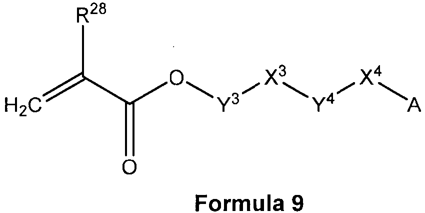 in which the variables have the following meanings:
A an aromatic group with 6 to 15 carbon atoms or a bicyclic or tricyclic aliphatic group with 7 to 10 carbon atoms, wherein A is unsubstituted or substituted by one or more C₁-C₅ alkyl groups, C₁-C₅ alkoxy groups and/or chlorine atoms;
R²⁸ hydrogen or methyl;
X³, X⁴ independently of each other is in each case are absent or an ether, ester or urethane group, wherein X³ is absent if Y³ is absent and wherein X⁴ is absent if Y⁴ is absent;
Y³, Y⁴ independently of each other is in each case absent or a branched or preferably linear aliphatic hydrocarbon radical with 1 to 10 carbon atoms, which is uninterrupted or interrupted by 1 to 3 oxygen atoms;
preferably a monomer selected from the group consisting of 2-[(benzyloxycarbonyl)-amino]-ethyl (meth)acrylate, 2-[(benzylcarbamoyl)-oxy]-ethyl (meth)-acrylate, and 2-(p-cumylphenoxy)-ethyl (meth)acrylate, 2-hydroxy-3-phenoxypropyl (meth)acrylate, 2-[(benzyloxycarbonyl)amino]-ethyl (meth)acrylate, 1-phenoxypropan-2-yl (meth)acrylate, 2-(benzyloxy)ethyl (meth)acrylate, 3-phenoxybenzyl (meth)acrylate, phenoxypropyl (meth)acrylate, 2-benzyloxyethyl (meth)acrylate, 2-benzoyloxyethyl (meth)acrylate, 2-(meth)acryloyloxybenzoic acid methyl ester, 2-phenylethyl (meth)acrylate, 2-(p-cumylphenoxy)ethyl (meth)acrylate, preferably 2-phenoxyethyl (meth)acrylate, 2-(o-biphenyloxy)-ethyl (meth)acrylate, tricyclodecane (meth)acrylate, tricyclodecane methanol (meth)acrylate and isobornyl (meth)acrylate, and mixtures thereof.

9. The material according to claim 7 or 8, which comprises as component (b) at least one urethane dimethacrylate, preferably a urethane dimethacrylate according to Formula 12 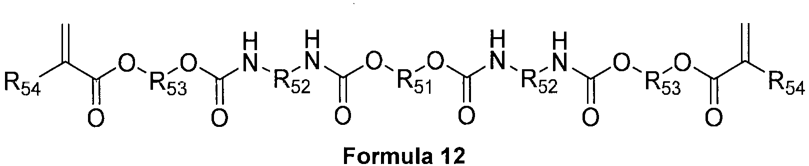 wherein:
R⁵¹ is a branched or preferably linear C₂-C₁₂ alkanediyl group, preferably a C₂-C₁₀ alkanediyl group, and more preferably a C₂-C₈ alkanediyl group, that can be interrupted by one or more O atoms and/or -C(O)O- groups;
or
a C₄-C₁₆ cycloaliphatic or tricyclic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and that can be interrupted by one or more O atoms and/or -C(O)O- groups;
or
a combination of the branched or the linear C₁-C₁₆ alkanediyl group and the C₄-C₁₆ cycloaliphatic group;
or
a C₇₋C₃₀-alkylaryl group that can be interrupted by one or more O atoms and/or C(O)O- groups and/or can be unsubstituted or substituted by one or more substituents selected from branched or preferably linear C₁-C₂₀ alkyl groups, branched or preferably linear -O-C₁-C₁₂ alkoxy groups, ester groups -C(O)O-R³, where R⁸ is a branched or preferably linear C₁-C₂₀ alkyl group and/or acyl groups -C(O)-R⁴, where R⁴ is a branched or preferably linear C₁-C₂₀ alkyl group;
R⁵² is a linear, branched or cyclic aliphatic or an aromatic or a combined aliphatic and aromatic group containing 6 to 15 carbon atoms;
R⁵³ is a branched or preferably linear C₂-C₁₀ alkanediyl group, preferably a C₂-C₁₈ alkanediyl group, and more preferably a C₂-C₆ alkanediyl group, that can be interrupted by one or more O atoms and/or -C(O)O- groups;
R⁵⁴ is H or CH₃.

10. The material according to any one of claims 7 to 9, which comprises as component (e) at least one photoinitiator, which is selected from monoacyltrialkyl-, diacyldialkyl-, tetraacylgermanium and tetraacylstannanes, preferably benzoyltrimethylgermanium, dibenzoyldiethylgermanium, bis(4-methoxybenzoyl)diethylgermanium, tetrakis(2-methylbenzoyl)germane or tetrakis(mesitoyl)stannane, more preferably acyl- or bisacylphosphine oxides, most preferably 2,4,6-trimethylbenzoyldiphenylphosphine oxide, ethyl (2,4,6-trimethylbenzoyl) phenylphosphinateand, bis(2,4,6-trimethylbenzoyl)phenylphosphine oxide, (2,4-dimethoxy-6-methylbenzoyl)diphenylphosphine oxide, (2,4,6-trimethoxybenzoyl)diphenylphosphine oxide or a mixture thereof.

11. The dental material according to any one of claims 6 to 10, which additionally comprises:
(f) 0 to 20 wt.%, preferably of from 0 to 10 wt.% and more preferably 0 wt.% of one or more fillers; and/or
(g) 0 to 1.0 wt.%, preferably of from 0.01 to 0.5 wt.% and more preferably of from 0.01 to 0.2 wt.% of one or more UV absorbers, preferably bumetrizole, 2,2',4,4'-tetrahydroxybenzophenone; and/or
(h) 0 to 0.1 wt.%, preferably of from 0.001 to 0.05 wt.% and more preferably of from 0.002 to 0.02 wt.% of one or more optical brighteners, 2,5-bis(5-tert-butyl-benzoxazol-2-yl)thiophene, fluorescent agents in the form of terephthalic acid derivatives,
whereby all percentages refer to the total weight of the dental material.

12. The dental material according to any one of claims 6 to 10, which comprises:
(a) 30 to 70 wt.%, preferably 30 to 65 wt.% and more preferably 40 to 60 wt.% of at least one monofunctional (meth)acrylate selected from 2-phenoxyethyl (meth)acrylate, 2-(o-biphenyloxy)ethyl (meth)acrylate, tricyclodecane (meth)acrylate, tricyclodecane methanol (meth)acrylate or isobornyl (meth)acrylate;
(b) 20 to 65 wt.%, preferably 25 to 60 wt.% and more preferably 30 to 55 wt.% of a urethane di(meth)acrylate according to Formula 12 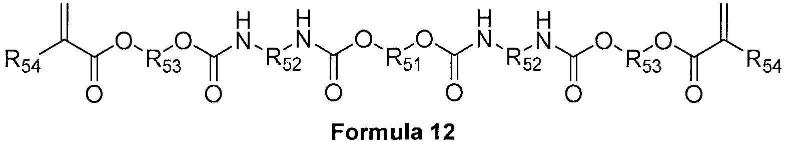 wherein:
R⁵¹ is a branched or preferably linear C₂-C₁₂ alkanediyl group, preferably a C₂-C₁₀ alkanediyl group, and more preferably a C₂-C₈ alkanediyl group, that can be interrupted by one or more O atoms and/or -C(O)O- groups;
or
a C₄-C₁₆ cycloaliphatic or tricyclic group that can be unsubstituted or substituted by one or more substituents selected from branched or preferably linear C₁-C₂₀ alkyl groups, preferably C₁-C₁₂ alkyl groups, most preferably C₁-C₆ alkyl groups, and that can be interrupted by one or more O atoms and/or -C(O)O- groups;
or
a combination of the branched or the linear C₁-C₁₆ alkanediyl group and the C₄-C₁₆ cycloaliphatic group;
or
a C₇-C₃₀-alkylaryl group that can be interrupted by one or more O atoms and/or C(O)O- groups and/or can be unsubstituted or substituted by one or more substituents selected from branched or preferably linear C₁-C₂₀ alkyl groups, branched or preferably linear -O-C₁-C₁₂ alkoxy groups, ester groups -C(O)O-R³, where R⁸ is a branched or preferably linear C₁-C₂₀ alkyl group and/or acyl groups -C(O)-R⁴, where R⁴ is a branched or preferably linear C₁-C₂₀ alkyl group;
R⁵² is a linear, branched or cyclic aliphatic or an aromatic or a combined aliphatic and aromatic group containing 6 to 15 carbon atoms;
R⁵³ is a branched or preferably linear C₂-C₁₀ alkanediyl group, preferably a C₂-C₁₈ alkanediyl group, and more preferably a C₂-C₆ alkanediyl group, that can be interrupted by one or more O atoms and/or -C(O)O- groups;
R⁵⁴ is CH₃;
(d) 1 to 15 wt.%, preferably 2 to 13 wt.% und more preferably 3 to 10 wt.% of the at least one block copolymer according to any one of claims 1 to 4,
(e) 0.1 to 5.0 wt.%, preferably 0.2 to 4.0 wt.% and more preferably 0.3 to 3.0 wt.% of at least one photoinitiator.

13. A process for the production of dental shaped parts in which
(i) a virtual image of the tooth situation is created by direct or indirect digitization of the tooth to be restored or of the teeth to be restored on a computer,
(ii) then a model of the dental restoration or prosthesis is constructed on the computer on the basis of this image,
(iii) a material in accordance with one of claims 5 to 12 is then polymerized in layers by selective light irradiation in order to form the dental restoration,
(iv) the workpiece is then cleaned, optionally by treatment with a solvent, and
(v) the workpiece is then optionally further cured by irradiating with light and/or by heating to a temperature above 50°C.

14. Use of a block copolymer according to any one of claims 1 to 4, for increasing the fracture toughness of a radically polymerizable composition.

15. Use of a material in accordance with one of claims 6 to 12 as dental material or for the production or repair of dental shaped parts.
